(19) Europäisches Patentamt
European Patent Office
Office européen des brevets

(11) EP 2 628 785 B1

# (12) EUROPEAN PATENT SPECIFICATION

(45) Date of publication and mention of the grant of the patent:
**18.05.2016 Bulletin 2016/20**

(51) Int Cl.:
***C11D 3/386*** *(2006.01)* ***C12N 9/52*** *(2006.01)*

(21) Application number: **12155909.0**

(22) Date of filing: **17.02.2012**

## (54) Detergent compositions comprising subtilase variants

Reinigungsmittelzusammensetzungen mit Subtilasevarianten

Compositions détergentes comprenant des variants de subtilase

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**21.08.2013 Bulletin 2013/34**

(73) Proprietor: **Henkel AG & Co. KGaA**
**40589 Düsseldorf (DE)**

(72) Inventors:
- **Besenmatter, Werner**
  **2860 Soeborg (DK)**
- **Benie, Astrid**
  **3500 Vaerloese (DK)**
- **Malten, Marco**
  **2400 Copenhagen NV (DK)**

(56) References cited:
**WO-A1-2011/036264 US-A1- 2011 251 073**

- **DATABASE Geneseq [Online] 1 September 2011 (2011-09-01), "Subtilisin BPN' mutant G97T-G128S-Y217D.", XP002678205, retrieved from EBI accession no. GSP:AZL30069 Database accession no. AZL30069 & WO 2011/072117 A1 (PROCTER & GAMBLE [US]; SOUTER PHILIP FRANK [GB]; WARD GLENN STEVEN [GB]) 16 June 2011 (2011-06-16)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 628 785 B1

Printed by Jouve, 75001 PARIS (FR)

SEQUENCE LISTING

<110> Henkel AG & Co. KGaA

<120> Detergent compositions comprising subtilase variants

<130> PT031293

<160> 4

<170> PatentIn version 3.3

<210> 1
<211> 1149
<212> DNA
<213> Bacillus amyloliquefaciens

<220>
<221> CDS
<222> (1)..(1146)

<220>
<221> sig_peptide
<222> (1)..(90)

<220>

<221> mat_peptide
<222> (322) .. (1146)

<400> 1

```
atg aga ggc  aaa aaa gta tgg atc  agt ttg ctg ttt gct tta gcg tta       48
Met Arg Gly  Lys Lys Val Trp Ile  Ser Leu Leu Phe Ala Leu Ala Leu
        -105                -100                 -95

atc ttt acg atg gcg ttc ggc agc aca tcc tct gcc cag gcg gca ggg         96
Ile Phe Thr Met Ala Phe Gly Ser Thr Ser Ser Ala Gln Ala Ala Gly
    -90                 -85                 -80

aaa tca aac ggg gaa aag aaa tat att gtc ggg ttt aaa cag aca atg        144
Lys Ser Asn Gly Glu Lys Lys Tyr Ile Val Gly Phe Lys Gln Thr Met
-75                 -70                 -65                 -60

agc acg atg agc gcc gct aag aag aaa gat gtc att tct gaa aaa ggc        192
Ser Thr Met Ser Ala Ala Lys Lys Lys Asp Val Ile Ser Glu Lys Gly
                -55                 -50                 -45

ggg aaa gtg caa aag caa ttc aaa tat gta gac gca gct tca gct aca        240
Gly Lys Val Gln Lys Gln Phe Lys Tyr Val Asp Ala Ala Ser Ala Thr
            -40                 -35                 -30

tta aac gaa aaa gct gta aaa gaa ttg aaa aaa gac ccg agc gtc gct        288
Leu Asn Glu Lys Ala Val Lys Glu Leu Lys Lys Asp Pro Ser Val Ala
        -25                 -20                 -15

tac gtt gaa gaa gat cac gta gca cat gcg tac gcg cag tcc gtg cct        336
Tyr Val Glu Glu Asp His Val Ala His Ala Tyr Ala Gln Ser Val Pro
    -10                 -5                  -1  1               5

tac ggc gta tca caa att aaa gcc cct gct ctg cac tct caa ggc tac        384
Tyr Gly Val Ser Gln Ile Lys Ala Pro Ala Leu His Ser Gln Gly Tyr
                10                  15                  20

act gga tca aat gtt aaa gta gcg gtt atc gac agc ggt atc gat tct        432
Thr Gly Ser Asn Val Lys Val Ala Val Ile Asp Ser Gly Ile Asp Ser
```

```
            25                  30                  35

tct cat cct gat tta aag gta gca ggc gga gcc agc atg gtt cct tct      480
Ser His Pro Asp Leu Lys Val Ala Gly Gly Ala Ser Met Val Pro Ser
        40                  45                  50

gaa aca aat cct ttc caa gac aac aac tct cac gga act cac gtt gcc      528
Glu Thr Asn Pro Phe Gln Asp Asn Asn Ser His Gly Thr His Val Ala
    55                  60                  65

ggc aca gtt gcg gct ctt aat aac tca atc ggt gta tta ggc gtt gcg      576
Gly Thr Val Ala Ala Leu Asn Asn Ser Ile Gly Val Leu Gly Val Ala
70                  75                  80                  85

cca agc gca tca ctt tac gct gta aaa gtt ctc ggt gct gac ggt tcc      624
Pro Ser Ala Ser Leu Tyr Ala Val Lys Val Leu Gly Ala Asp Gly Ser
                90                  95                  100

ggc caa tac agc tgg atc att aac gga atc gag tgg gcg atc gca aac      672
Gly Gln Tyr Ser Trp Ile Ile Asn Gly Ile Glu Trp Ala Ile Ala Asn
            105                 110                 115

aat atg gac gtt att aac atg agc ctc ggc gga cct tct ggt tct gct      720
Asn Met Asp Val Ile Asn Met Ser Leu Gly Gly Pro Ser Gly Ser Ala
        120                 125                 130

gct tta aaa gcg gca gtt gat aaa gcc gtt gca tcc ggc gtc gta gtc      768
Ala Leu Lys Ala Ala Val Asp Lys Ala Val Ala Ser Gly Val Val Val
    135                 140                 145

gtt gcg gca gcc ggt aac gaa ggc act tcc ggc agc tca agc aca gtg      816
Val Ala Ala Ala Gly Asn Glu Gly Thr Ser Gly Ser Ser Ser Thr Val
150                 155                 160                 165

ggc tac cct ggt aaa tac cct tct gtc att gca gta ggc gct gtt gac      864
Gly Tyr Pro Gly Lys Tyr Pro Ser Val Ile Ala Val Gly Ala Val Asp
                170                 175                 180

agc agc aac caa aga gca tct ttc tca agc gta gga cct gag ctt gat      912
Ser Ser Asn Gln Arg Ala Ser Phe Ser Ser Val Gly Pro Glu Leu Asp
            185                 190                 195

gtc atg gca cct ggc gta tct atc caa agc acg ctt cct gga aac aaa      960
Val Met Ala Pro Gly Val Ser Ile Gln Ser Thr Leu Pro Gly Asn Lys
        200                 205                 210

tac ggg gcg tac aac ggt acg tca atg gca tct ccg cac gtt gcc gga     1008
Tyr Gly Ala Tyr Asn Gly Thr Ser Met Ala Ser Pro His Val Ala Gly
    215                 220                 225

gcg gct gct ttg att ctt tct aag cac ccg aac tgg aca aac act caa     1056
Ala Ala Ala Leu Ile Leu Ser Lys His Pro Asn Trp Thr Asn Thr Gln
230                 235                 240                 245

gtc cgc agc agt tta gaa aac acc act aca aaa ctt ggt gat tct ttc     1104
Val Arg Ser Ser Leu Glu Asn Thr Thr Thr Lys Leu Gly Asp Ser Phe
                250                 255                 260

tac tat gga aaa ggg ctg atc aac gta cag gcg gca gct cag taa         1149
Tyr Tyr Gly Lys Gly Leu Ile Asn Val Gln Ala Ala Ala Gln
            265                 270                 275
```

<210> 2

<211> 382
<212> PRT
<213> Bacillus amyloliquefaciens

<400> 2

Met Arg Gly Lys Lys Val Trp Ile Ser Leu Leu Phe Ala Leu Ala Leu
-105 -100 -95

Ile Phe Thr Met Ala Phe Gly Ser Thr Ser Ser Ala Gln Ala Ala Gly
-90 -85 -80

Lys Ser Asn Gly Glu Lys Lys Tyr Ile Val Gly Phe Lys Gln Thr Met
-75 -70 -65 -60

Ser Thr Met Ser Ala Ala Lys Lys Lys Asp Val Ile Ser Glu Lys Gly
-55 -50 -45

Gly Lys Val Gln Lys Gln Phe Lys Tyr Val Asp Ala Ala Ser Ala Thr
-40 -35 -30

Leu Asn Glu Lys Ala Val Lys Glu Leu Lys Lys Asp Pro Ser Val Ala
-25 -20 -15

Tyr Val Glu Glu Asp His Val Ala His Ala Tyr Ala Gln Ser Val Pro
-10 -5 -1 1 5

Tyr Gly Val Ser Gln Ile Lys Ala Pro Ala Leu His Ser Gln Gly Tyr
10 15 20

Thr Gly Ser Asn Val Lys Val Ala Val Ile Asp Ser Gly Ile Asp Ser
25 30 35

Ser His Pro Asp Leu Lys Val Ala Gly Gly Ala Ser Met Val Pro Ser
40 45 50

Glu Thr Asn Pro Phe Gln Asp Asn Asn Ser His Gly Thr His Val Ala
55 60 65

Gly Thr Val Ala Ala Leu Asn Asn Ser Ile Gly Val Leu Gly Val Ala
70 75 80 85

Pro Ser Ala Ser Leu Tyr Ala Val Lys Val Leu Gly Ala Asp Gly Ser
90 95 100

Gly Gln Tyr Ser Trp Ile Ile Asn Gly Ile Glu Trp Ala Ile Ala Asn
105 110 115

Asn Met Asp Val Ile Asn Met Ser Leu Gly Gly Pro Ser Gly Ser Ala
120 125 130

Ala Leu Lys Ala Ala Val Asp Lys Ala Val Ala Ser Gly Val Val Val
135 140 145

Val Ala Ala Ala Gly Asn Glu Gly Thr Ser Gly Ser Ser Ser Thr Val
150 155 160 165

Gly Tyr Pro Gly Lys Tyr Pro Ser Val Ile Ala Val Gly Ala Val Asp
170 175 180

Ser Ser Asn Gln Arg Ala Ser Phe Ser Ser Val Gly Pro Glu Leu Asp
185 190 195

Val Met Ala Pro Gly Val Ser Ile Gln Ser Thr Leu Pro Gly Asn Lys
200 205 210

Tyr Gly Ala Tyr Asn Gly Thr Ser Met Ala Ser Pro His Val Ala Gly
215 220 225

Ala Ala Ala Leu Ile Leu Ser Lys His Pro Asn Trp Thr Asn Thr Gln
230 235 240 245

Val Arg Ser Ser Leu Glu Asn Thr Thr Thr Lys Leu Gly Asp Ser Phe
250 255 260

Tyr Tyr Gly Lys Gly Leu Ile Asn Val Gln Ala Ala Ala Gln
265 270 275

<210> 3
<211> 1143
<212> DNA
<213> Bacillus lentus

<220>
<221> CDS
<222> (1)..(1140)

<220>
<221> sig_peptide
<222> (1) .. (81)

<220>
<221> mat_peptide
<222> (334)..(1140)

<400> 3

```
atg aag  aaa ccg ttg ggg aaa  att gtc gca agc acc  gca cta ctc      45
Met Lys  Lys Pro Leu Gly Lys  Ile Val Ala Ser Thr  Ala Leu Leu
    -110                 -105                 -100

att tct gtt gct ttt agt tca tcg atc gca tcg gct gct gaa gaa gca     93
Ile Ser Val Ala Phe Ser Ser Ser Ile Ala Ser Ala Ala Glu Glu Ala
    -95                 -90                 -85

aaa gaa aaa tat tta att ggc ttt aat gag cag gaa gct gtc agt gag    141
Lys Glu Lys Tyr Leu Ile Gly Phe Asn Glu Gln Glu Ala Val Ser Glu
```

-80 -75 -70 -65

ttt gta gaa caa gta gag gca aat gac gag gtc gcc att ctc tct gag 189
Phe Val Glu Gln Val Glu Ala Asn Asp Glu Val Ala Ile Leu Ser Glu
-60 -55 -50

gaa gag gaa gtc gaa att gaa ttg ctt cat gaa ttt gaa acg att cct 237
Glu Glu Glu Val Glu Ile Glu Leu Leu His Glu Phe Glu Thr Ile Pro
-45 -40 -35

gtt tta tcc gtt gag tta agc cca gaa gat gtg gac gcg ctt gaa ctc 285
Val Leu Ser Val Glu Leu Ser Pro Glu Asp Val Asp Ala Leu Glu Leu
-30 -25 -20

gat cca gcg att tct tat att gaa gag gat gca gaa gta acg aca atg 333
Asp Pro Ala Ile Ser Tyr Ile Glu Glu Asp Ala Glu Val Thr Thr Met
-15 -10 -5 -1

gcg caa tcg gta cca tgg gga att agc cgt gtg caa gcc cca gct gcc 381
Ala Gln Ser Val Pro Trp Gly Ile Ser Arg Val Gln Ala Pro Ala Ala
1 5 10 15

cat aac cgt gga ttg aca ggt tct ggt gta aaa gtt gct gtc ctc gat 429
His Asn Arg Gly Leu Thr Gly Ser Gly Val Lys Val Ala Val Leu Asp
20 25 30

aca ggg ata tcc act cat cca gat cta aat att cgt ggt ggc gca agc 477
Thr Gly Ile Ser Thr His Pro Asp Leu Asn Ile Arg Gly Gly Ala Ser
35 40 45

ttt gta cca ggg gaa ccg tcg act caa gat ggg aat ggg cat ggc acg 525
Phe Val Pro Gly Glu Pro Ser Thr Gln Asp Gly Asn Gly His Gly Thr
50 55 60

cat gtg gcc ggg acg atc gct gct tta aac aat tcg att ggc gtt ctt 573
His Val Ala Gly Thr Ile Ala Ala Leu Asn Asn Ser Ile Gly Val Leu
65 70 75 80

ggc gta gct cct agc gct gag cta tac gct gtt aaa gtc cta ggg gcg 621
Gly Val Ala Pro Ser Ala Glu Leu Tyr Ala Val Lys Val Leu Gly Ala
85 90 95

agc ggt tca ggt tcg gtc agc tcg att gcc caa gga ttg gaa tgg gca 669
Ser Gly Ser Gly Ser Val Ser Ser Ile Ala Gln Gly Leu Glu Trp Ala
100 105 110

ggg aac aat ggc atg cac gtt gct aat ttg agt tta gga agc cct tcg 717
Gly Asn Asn Gly Met His Val Ala Asn Leu Ser Leu Gly Ser Pro Ser
115 120 125

cca agt gcc aca ctc gag caa gct gtt aat agc gcg act tct aga ggc 765
Pro Ser Ala Thr Leu Glu Gln Ala Val Asn Ser Ala Thr Ser Arg Gly
130 135 140

gtt ctt gtt gta gcg gca tct ggg aat tca ggt gca ggc tca atc agc 813
Val Leu Val Val Ala Ala Ser Gly Asn Ser Gly Ala Gly Ser Ile Ser
145 150 155 160

tat ccg gcg cgc tat gcg aac gca atg gca gtc gga gct act gat caa 861
Tyr Pro Ala Arg Tyr Ala Asn Ala Met Ala Val Gly Ala Thr Asp Gln
165 170 175

aac aac aac cgc gct agc ttt tca cag tat ggc gca ggc ctt gac att 909
Asn Asn Asn Arg Ala Ser Phe Ser Gln Tyr Gly Ala Gly Leu Asp Ile
180 185 190

```
gtc gca ccc ggg gta aac gtg cag agc aca tac cca ggt tca aca tat       957
Val Ala Pro Gly Val Asn Val Gln Ser Thr Tyr Pro Gly Ser Thr Tyr
        195                     200                     205

gcc agc tta aac ggt aca tcg atg gct act cct cat gtt gca ggt gcg      1005
Ala Ser Leu Asn Gly Thr Ser Met Ala Thr Pro His Val Ala Gly Ala
    210                     215                     220

gcc gcc ctt gtt aaa caa aag aac cca tct tgg tct aat gta caa att      1053
Ala Ala Leu Val Lys Gln Lys Asn Pro Ser Trp Ser Asn Val Gln Ile
225                     230                     235                 240

cga aat cat cta aag aat acg gca act agt tta gga agc acg aac ttg      1101
Arg Asn His Leu Lys Asn Thr Ala Thr Ser Leu Gly Ser Thr Asn Leu
                245                     250                     255

tat gga agc gga ctt gtt aac gca gaa gcg gca acg cgt taa              1143
Tyr Gly Ser Gly Leu Val Asn Ala Glu Ala Ala Thr Arg
            260                     265
```

<210> 4
<211> 380
<212> PRT
<213> Bacillus lentus

<400> 4

```
Met Lys Lys Pro Leu Gly Lys Ile Val Ala Ser Thr Ala Leu Leu
    -110                -105                -100

Ile Ser Val Ala Phe Ser Ser Ser Ile Ala Ser Ala Ala Glu Glu Ala
    -95                 -90                 -85

Lys Glu Lys Tyr Leu Ile Gly Phe Asn Glu Gln Glu Ala Val Ser Glu
-80                 -75                 -70                 -65

Phe Val Glu Gln Val Glu Ala Asn Asp Glu Val Ala Ile Leu Ser Glu
                -60                 -55                 -50

Glu Glu Glu Val Glu Ile Glu Leu Leu His Glu Phe Glu Thr Ile Pro
            -45                 -40                 -35

Val Leu Ser Val Glu Leu Ser Pro Glu Asp Val Asp Ala Leu Glu Leu
        -30                 -25                 -20

Asp Pro Ala Ile Ser Tyr Ile Glu Glu Asp Ala Glu Val Thr Thr Met
    -15                 -10                 -5                  -1

Ala Gln Ser Val Pro Trp Gly Ile Ser Arg Val Gln Ala Pro Ala Ala
1               5                   10                  15

His Asn Arg Gly Leu Thr Gly Ser Gly Val Lys Val Ala Val Leu Asp
            20                  25                  30
```

Thr Gly Ile Ser Thr His Pro Asp Leu Asn Ile Arg Gly Gly Ala Ser
35 40 45

Phe Val Pro Gly Glu Pro Ser Thr Gln Asp Gly Asn Gly His Gly Thr
50 55 60

His Val Ala Gly Thr Ile Ala Ala Leu Asn Asn Ser Ile Gly Val Leu
65 70 75 80

Gly Val Ala Pro Ser Ala Glu Leu Tyr Ala Val Lys Val Leu Gly Ala
85 90 95

Ser Gly Ser Gly Ser Val Ser Ser Ile Ala Gln Gly Leu Glu Trp Ala
100 105 110

Gly Asn Asn Gly Met His Val Ala Asn Leu Ser Leu Gly Ser Pro Ser
115 120 125

Pro Ser Ala Thr Leu Glu Gln Ala Val Asn Ser Ala Thr Ser Arg Gly
130 135 140

Val Leu Val Val Ala Ala Ser Gly Asn Ser Gly Ala Gly Ser Ile Ser
145 150 155 160

Tyr Pro Ala Arg Tyr Ala Asn Ala Met Ala Val Gly Ala Thr Asp Gln
165 170 175

Asn Asn Asn Arg Ala Ser Phe Ser Gln Tyr Gly Ala Gly Leu Asp Ile
180 185 190

Val Ala Pro Gly Val Asn Val Gln Ser Thr Tyr Pro Gly Ser Thr Tyr
195 200 205

Ala Ser Leu Asn Gly Thr Ser Met Ala Thr Pro His Val Ala Gly Ala
210 215 220

Ala Ala Leu Val Lys Gln Lys Asn Pro Ser Trp Ser Asn Val Gln Ile
225 230 235 240

Arg Asn His Leu Lys Asn Thr Ala Thr Ser Leu Gly Ser Thr Asn Leu
245 250 255

Tyr Gly Ser Gly Leu Val Asn Ala Glu Ala Ala Thr Arg
260 265

## Description

### Reference to a Sequence Listing

This application contains a Sequence Listing in computer readable form, which is incorporated herein by reference.

### Background of the Invention

### Field of the Invention

The present invention relates to protease containing detergent compositions, especially detergent compositions comprising subtilisin variants exhibiting alterations relative to the parent subtilisin in one or more properties including: wash performance, thermal stability, storage stability or catalytic activity. Further, the present invention relates to methods of producing said detergent compositions and to the use of said detergent compositions in cleaning applications.

### Description of the Related Art

In the detergent industry enzymes have for more than 30 years been implemented in washing formulations. Enzymes used in such formulations comprise proteases, lipases, amylases, cellulases, mannosidases as well as other enzymes or mixtures thereof. Commercially the most important enzymes are proteases.

An increasing number of commercially used proteases are protein engineered variants of naturally occurring wild type proteases, e.g. Everlase®, Relase®, Ovozyme®, Polarzyme®, Liquanase®, Liquanase Ultra® and Kannase® (Novozymes a/s), Purafast®, Purafect OXP®, FN3®, FN4® and Excellase® (Genencor International, Inc.) and BLAP (Figure 29, US5352604) (Henkel AG & Co. KGaA).

Further, a number of variants are described in the art, such as in WO 04/041979 (NOVOZYMES A/S) which describes subtilisin variants exhibiting alterations relative to the parent subtilisin in e.g. wash performance, thermal stability, storage stability or catalytic activity. The variants are suitable for use in e.g. cleaning or detergent compositions.

A number of useful subtilisin variants have been described many of which have provided improved activity, stability, and solubility in different detergents. WO9402618 describes subtilisin variants with improved storage stability. The substitutions L211X and N212Z wherein X is any amino acid except L and Z is any amino acid except N are mentioned together with several other mutations. However, no wash performance is shown for these variants and nothing is mentioned in relation to specific stains such as egg stains. It is well known in the art that egg stains are particularly difficult to completely remove and although several protease variants with improved performance on egg stains have been described, e.g. in WO0144452, there is still a need for detergent compositions with proteases which have high wash performance on various stains including egg stains.

It is therefore an object of the present invention to provide detergent compositions with variants of a protease with improved properties compared to its parent protease.

### Summary of the Invention

The present invention relates to a detergent composition comprising a subtilisin variant comprising the substitutions corresponding to Y217X and N218Z of SEQ ID NO: 2, wherein X and Z are selected from the group consisting of D or E and wherein the variant has an amino acid sequence which is at least 90 % identical to SEQ ID NO: 2 or 4. and wherein the number of alterations in the subtilisin variant is 1-20, e.g., 1-10 and 1-5, such as 1, 2, 3, 4, 5, 6, 7, 8, 9 or 10 alterations.

The present invention also relates to a method for producing a detergent composition which comprises the step of adding a subtilisin variant which was obtained by a method comprising introducing into a parent subtilisin the substitutions corresponding to Y217X and N218Z of SEQ ID NO: 2, wherein X and Z are selected from the group consisting of D or, E, and wherein the variant has an amino acid sequence which is at least 90 % identical to SEQ ID NO: 2 or 4, and wherein the number of alterations in the subtilisin variant is 1-20, e.g., 1-10 and 1-5, such as 1, 2, 3, 4, 5, 6, 7, 8, 9 or 10 alterations. The invention also relates to detergent compositions obtained by such method.

The invention further relates to the use of detergent compositions according to the invention in cleaning processes such as laundry and/or dish wash.

### Definitions

**Protease:** The term "protease" is defined herein as an enzyme that hydrolyses peptide bonds. It includes any

enzyme belonging to the EC 3.4 enzyme group (including each of the thirteen subclasses thereof). The EC number refers to Enzyme Nomenclature 1992 from NC-IUBMB, Academic Press, San Diego, California, including supplements 1-5 published in Eur. J. Biochem. 1994, 223, 1-5; Eur. J. Biochem. 1996, 232, 1-6; Eur. J. Biochem. 1996, 237, 1-5; Eur. J. Biochem. 1997, 250, 1-6; and Eur. J. Biochem. 1999, 264, 610-650; respectivety.

**Protease activity:** The term "protease activity" means a proteolytic activity (EC 3.4). Proteases of the invention are endopeptidases (EC 3.4.21). There are several protease activity types: The three main activity types are: trypsin-like where there is cleavage of amide substrates following Arg or Lys at P1, chymotrypsin-like where cleavage occurs following one of the hydrophobic amino acids at P1, and elastase-like with cleavage following an Ala at P1. For purposes of the present invention, protease activity is determined according to the procedure described in "Materials and Methods" below. The subtilisin variants of the present invention have at least 20%, *e.g.,* at least 40%, at least 50%, at least 60%, at least 70%, at least 80%, at least 90%, at least 95%, and at least 100% of the protease activity of the mature polypeptide of SEQ ID NO: 2 or 4.

**Improved property:** The term "improved property" means a characteristic associated with a variant that is improved compared to the parent or compared to a protease with SEQ ID NO: 2, or compared to a protease having the identical amino acid sequence of said variant but not having the alterations at one or more of said specified positions. Such improved properties include, but are not limited to wash performance, protease activity, thermal activity profile, thermostability, pH activity profile, pH stability, substrate/cofactor specificity, improved surface properties, substrate specificity, product specificity, increased stability e.g. chelator stability or solubility in the presence of pretreated biomass, improved stability under storage conditions, and chemical stability.

**Improved chemical stability:** The term "improved chemical stability" is defined herein as a variant enzyme displaying retention of enzymatic activity after a period of incubation in the presence of a chemical or chemicals, either naturally occurring or synthetic, which reduces the enzymatic activity of the parent enzyme. Improved chemical stability may also result in variants better able to catalyze a reaction in the presence of such chemicals. In a particular aspect of the invention the improved chemical stability is an improved stability in a detergent, in particular in a liquid detergent. The improved detergent stability is in particular an improved stability of the alpha amylase activity when an alpha amylase variant of the present invention is mixed into a liquid detergent formulation comprising a chelating agent, the liquid also includes gels or a paste. The liquid detergent formulation may refer to concentrated detergent which is added during a laundry or automated dish wash process or a dilute detergent such as a wash solution, i.e. an aqueous solution to which the concentrated detergent is added.

In the present invention liquid detergents are particular useful as liquid laundry detergents.

**Stability** The term "stability" includes storage stability and stability during use, e.g. during a wash process and reflects the stability of the protease as a function of time e.g. how much activity is retained when the protease is kept in solution in particular in a detergent solution. The stability is influenced by many factors e.g. pH, temperature, detergent composition e.g. amount of builder, surfactants etc.

**Improved wash performance:** The term "improved wash performance" is defined herein as a protease variant or a detergent composition comprising said protease variant displaying an alteration of the wash performance relative to the wash performance of the parent subtilisin or the corresponding detergent composition, relative to a protease with SEQ ID NO: 2 or the corresponding detergent composition, or relative to a protease having the identical amino acid sequence of said variant but not having the alterations at one or more of said specified positions or the corresponding detergent composition, e.g. by increased stain removal which is particularly preferred. The term "wash performance" includes wash performance in laundry but also e.g. in dish wash. The wash performance may be quantified as described under the definition of "wash performance" herein.

**Improved protease activity:** The term "improved protease activity" is defined herein as an altered protease activity (as defined above) of a protease variant displaying an alteration of the activity relative (or compared) to the activity of the parent subtilisin, or compared to a protease with SEQ ID NO: 2, or relative to a protease having the identical amino acid sequence of said variant but not having the alterations at one or more of said specified positions, by increased protein conversion.

**Mature polypeptide:** The term "mature polypeptide" means a polypeptide in its final form following translation and any post-translational modifications, such as N-terminal processing. C-terminal truncation, glycosylation, phosphorylation, etc. In one aspect, the mature polypeptide corresponds to the amino acid sequence with SEQ ID NO: 2 or SEQ ID NO: 4.

**Mature polypeptide coding sequence:** The term "mature polypeptide coding sequence" means a polynucleotide that encodes a mature polypeptide having protease activity. In one aspect, the mature polypeptide coding sequence is nucleotides 322 to 1146 of SEQ ID NO: 1 based on the SignalP (Nielsen et al., 1997, Protein Engineering 10: 1-6)] that predicts nucleotides 1 to 91 of SEQ ID NO: 1 encodes a signal peptide.

In one aspect, the mature polypeptide coding sequence is nucleotides 577 to 1140 of SEQ ID NO: 3 based on the signalP (Nielsen et al., 1997, Protein Engineering 10: 1-6)] that predicts nucleotides 1 to 81 of SEQ ID NO: 3 encodes a signal peptide.

**Parent:** The term "parent" means a protease to which an alteration is made to produce the enzyme variants of the present invention. Thus the parent is a protease having the identical amino acid sequence of said variant but not having the alterations at one or more e.g. two or more of said specified positions. It will be understood, that in the present context the expression "having identical amino acid sequence" relates to 100% sequence identity. The parent may be a naturally occurring (wild-type) polypeptide or a variant thereof. In a particular embodiment the parent is a protease with at least 60 % identity, such as at least 65%, at least 70%, at least 75%, at least 80%, at least 81%, at least 82%, at least 83%, at least 84%, at least 85%, at least 90%, at least 91%, at least 92%, at least 93%, at least 94%, at least 95%, at least 96%, at least 97%, at least 98%, at least 99%, or 100% identity to a polypeptide with SEQ ID NO: 2 or 4.

**Sequence Identity:** The relatedness between two amino acid sequences or between two nucleotide sequences is described by the parameter "sequence identity". For purposes of the present invention, the degree of sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 3.0.0 or later. The optional parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

**(Identical Residues x 100)/(Length of Alignment – Total Number of Gaps in Alignment)**

For purposes of the present invention, the degree of sequence identity between two deoxyribonucleotide sequences is determined using the Needleman-Wunsch algorithm (Needleman and Wunsch, 1970, *supra*) as implemented in the Needle program of the EMBOSS package (EMBOSS: The European Molecular Biology Open Software Suite, Rice et al., 2000, *supra*), preferably version 3.0.0 or later. The optional parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

**(Identical Deoxyribonucleotides x 100)/ (Length of Alignment – Total Number of Gaps in**

**Substantially pure variant:** The term "substantially pure variant" means a preparation that contains at most 10%, at most 8%, at most 6%, at most 5%, at most 4%, at most 3%, at most 2%, at most 1%, and at most 0.5% by weight of other polypeptide material with which it is natively or recombinantly associated. Preferably, the variant is at least 92% pure, e.g., at least 94% pure, at least 95% pure, at least 96% pure, at least 97% pure, at least 98% pure, at least 99%, at least 99.5% pure, and 100% pure by weight of the total polypeptide material present in the preparation. The variants of the present invention are preferably in a substantially pure form. This can be accomplished, for example, by preparing the variant by well known recombinant methods or by classical purification methods.

Variant: The term "variant" means a polypeptide having protease activity comprising an alteration, i.e., a substitution, insertion, and/or deletion, at one or more (or one or several) positions compared to its parent which is a protease having the Identical amino acid sequence of said variant but not having the alterations at one or more of said specified positions. A substitution means a replacement of an amino acid occupying a position with a different amino acid; a deletion means removal of an amino acid occupying a position; and an insertion means adding amino acids e.g. 1 to 10 amino acids, such as 9 amino acids, such as 8 amino acids, such as 7 amino acids, such as 6 amino acids, such as 5 amino acids, such as 4 amino acids, preferably 1-3 amino acids, more preferably 1-2 amino acids and most preferably two amino acids adjacent to an amino acid occupying a position.

**Wash performance:** The term "wash performance" is used as an enzyme's or detergent's ability to remove stains present on the object to be cleaned during e.g. wash, such as laundry or hard surface cleaning. The improvement in the wash performance may be quantified by calculating the so-called intensity value (Int) defined in AMSA assay as described in Materials and methods herein. See also the wash performance test in Example 2 herein. Further, the wash performance may be determined by the reference washing test described below.

**Wild-Type protease:** The term "wild-type protease" means a protease expressed by a naturally occurring organism, such as a bacterium, archaea, yeast, fungus, plant or animal found in nature. An example of a wild-type protease is BPN' i.e. SEQ ID NO: 2 or Savinase (SEQ ID NO: 4).

**Conventions for Designation of Variants**

For purposes of the present invention, the mature polypeptide disclosed in SEQ ID NO: 2 is used to determine the corresponding amino acid residue in another subtilisin. The amino acid sequence of another subtilisins is aligned with the mature polypeptide disclosed in (SEQ ID NO: 2, and based on the alignment; the amino acid position number corresponding to any amino acid residue in the mature polypeptide disclosed in SEQ ID NO: 2 is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix.

Identification of the corresponding amino acid residue in another subtilisin can be determined by an alignment of multiple polypeptide sequences using several computer programs including, but not limited to, MUSCLE (multiple sequence comparison by log-expectation; version 3.5 or later, Edgar, 2004, Nucleic Acids Research 32: 1792-1797), MAFFT (version 6.857 or later; Katoh and Kuma, 2002, Nucleic Acids Research 30: 3059-3066; Katoh et al., 2005, Nucleic Acids Research 33: 511-518; Katoh and Toh, 2007, Bioinformatics 23: 372-374; Katoh et al., 2009, Methods in Molecular Biology 537: 39-64; Katoh and Toh, 2010, Bioinformatics 26: 1899-1900), and EMBOSS EMMA employing ClustalW (1.83 or later; Thompson et al., 1994, Nucleic Acids Research 22: 4673-4680), using their respective default parameters.

When the other enzyme has diverged from the mature polypeptide of SEQ ID NO: 2 such that traditional sequence-based comparison fails to detect their relationship (Lindahl and Elofsson, 2000, J. Mol. Biol. 295: 613-615), other pairwise sequence comparison algorithms can be used. Greater sensitivity in sequence-based searching can be attained using search programs that utilize probabilistic representations of polypeptide families (profiles) to search databases. For example, the PSI-BLAST program generates profiles through an iterative database search process and is capable of detecting remote homologs (Atschul et al., 1997, Nucleic Acids Res. 25: 3389-3402). Even greater sensitivity can be achieved if the family or superfamily for the polypeptide has one or more representatives in the protein structure databases. Programs such as GenTHREADER (Jones, 1999, J. Mol. Biol. 287: 797-815; McGuffin and Jones, 2003, Bioinformatics 19: 874-881) utilize information from a variety of sources (PSI-BLAST, secondary structure prediction, structural alignment profiles, and solvation potentials) as input to a neural network that predicts the structural fold for a query sequence. Similarly, the method of Gough et al., 2000, J. Mol. Biol. 313: 903-919, can be used to align a sequence of unknown structure with the superfamily models present in the SCOP database. These alignments can in turn be used to generate homology models for the polypeptide, and such models can be assessed for accuracy using a variety of tools developed for that purpose.

For proteins of known structure, several tools and resources are available for retrieving and generating structural alignments. For example the SCOP superfamilies of proteins have been structurally aligned, and those alignments are accessible and downloadable. Two or more protein structures can be aligned using a variety of algorithms such as the distance alignment matrix (Holm and Sander, 1998, Proteins 33: 88-96) or combinatorial extension (Shindyalov and Bourne, 1998, Protein Engineering 11: 739-747), and implementation of these algorithms can additionally be utilized to query structure databases with a structure of interest in order to discover possible structural homologs (*e.g.*, Holm and Park, 2000, Bioinformatics 16: 566-567).

In describing the variants of the present invention, the nomenclature described below is adapted for ease of reference. The accepted IUPAC single letter or three letter amino acid abbreviation is employed.

Substitutions. For an amino acid substitution, the following nomenclature is used: Original amino acid, position, substituted amino acid. Accordingly, the substitution of threonine at position 226 with alanine is designated as "Thr226Ala" or "T226A". Multiple mutations are separated by addition marks ("+"), *e.g.*, "Gly205Arg + Ser411Phe" or "G205R + S411 F", representing substitutions at positions 205 and 411 of glycine (G) with arginine (R) and serine (S) with phenylalanine (F), respectively.

Deletions. For an amino acid deletion, the following nomenclature is used: Original amino acid, position, *. Accordingly, the deletion of glycine at position 195 is designated as "Gly195*" or "G195*". Multiple deletions are separated by addition marks ("+"), *e.g.*, "Gly195* + Ser411*" or "G195* + S411*".

Insertions. For an amino acid insertion, the following nomenclature is used: Original amino acid, position, original amino acid, inserted amino acid. Accordingly the insertion of lysine after glycine at position 195 is designated "Gly195GlyLys" or "G195GK". An insertion of multiple amino acids is designated [Original amino acid, position, original amino acid, inserted amino acid #1, inserted amino acid #2; etc.]. For example, the insertion of lysine and alanine after glycine at position 195 is indicated as "Gly195GlyLysAla" or "G195GKA".

In such cases the inserted amino acid residue(s) are numbered by the addition of lower case letters to the position number of the amino acid residue preceding the inserted amino acid residue(s). In the above example, the sequence would thus be:

| Parent: | Variant: |
|---|---|
| 195 | 195 195a 195b |
| G | G - K - A |

**[0037]** - Multiple alterations. Variants comprising multiple alterations are separated by addition marks ("+"), *e.g.*, "Arg170Tyr+Gly195Glu" or "R170Y+G195E" representing a substitution of arginine and glycine at positions 170 and 195 with tyrosine and glutamic acid, respectively.

Different alterations. Where different alterations can be introduced at a position, the different alterations are separated by a comma, *e.g.*, "Arg170Tyr,Glu" represents a substitution of arginine at position 170 with tyrosine or glutamic acid. Thus, "Tyr167Gly,Ala + Arg170Gly,Ala" designates the following variants:

"Tyr167Gly+Arg170Gly", "Tyr167Gly+Arg170Ala", "Tyr167Ala+Arg170Gly", and "Tyr167Ala+Arg170Ala".

Numbering of amino acid positions/residues

If nothing else is mentioned the amino acid numbering used herein correspond to that of the subtilase BPN' (BASBPN) sequence. For further description of the BPN' sequence, see SEQ ID NO:2 or Siezen et al., Protein Engng. 4 (1991) 719-737.

In the following the term "corresponding to" is to be understood as corresponding to a position of SEQ ID NO: 2, i.e. the numbering throughout the document is according to BPN'.

**Detailed Description of the Invention**

The inventors have found that changing the overall charge of in the positions corresponding to position 217 or 218 of SEQ ID NO: 2 resulted in variants with increased wash performance in particular on egg stains, therefore making such variants particularly applicable for detergent compositions, Increasing the negative charge at both positions corresponding to position 217 and 218 i.e. by substitution the amino acid occupying position 217 and the amino acid occupying position 218 with an amino acid which is more negatively charged resulted in protease variants with improved properties compared to its parent e.g. compared to SEQ ID NO: 2, which are advantageously applicable in detergent compositions. In particular the variants have increased the wash performance compared to a parent with SEQ ID NO: 2, SEQ ID NO: 2 with the Y217L mutation or compared to a parent with a single charge change in either position 217 or 218. Though a single substitution at either position 217 or 218 with an amino acid more charged than the substituted amino acid may provide a beneficial effect on wash performance this effect was more pronounced when both the positions 217 and 218 were made more negatively charged compared to SEQ ID NO: 2 i.e. by substituting the amino acids corresponding to 217 and 218 of SEQ ID NO: 2 in the parent protease with either the negatively charged amino acids D, E. The inventors found that increased wash performance is obtained when the substitutions lead to an overall change in charge at the two positions corresponding to position 217 ad 218 of SEQ ID NO: 2. Thus variants with the same charge at position 217 and 218 e.g. a negative charge on both positions such as Y217E+N218D showed increased performance compared the parent, to a protease with SEQ ID NO: 2 or relative to a protease having the identical amino acid sequence of said variant but not having the alterations at one or more of said specified positions. Making the protease either more negative charged at positions corresponding to position 217 or 218 of SEQ ID NO: 2 results in protease variants which are more hydrophilic. Thus according to another embodiment, the protease variants in detergent compositions according to the invention are more hydrophilic at the positions corresponding to positions 217 and 218 of SEQ ID NO: 2 compared the parent, to a protease with SEQ ID NO: 2 or relative to a protease having the identical amino acid sequence of said variant but not having the alterations at one or more of said specified positions.

Thus one aspect of the invention relates to detergent compositions comprising subtilisin variants comprising any of the substitutions corresponding to Y217D, Y217E, at least 90%, at least 91%, at least 92%, at least 93%, at least 94%at least 95% identity, at least 96%, at least 97%, at least 98%, or at least 99%, but less than 100%, sequence identity to the mature polypeptide of SEQ ID NO: 2 or 4, and wherein the number of alterations in the subtilisin variant is 1-20, *e.g*, 1-10 and 1-5, such as 1, 2, 3, 4, 5, 6, 7, 8, 9 or 10 alterations.

In one embodiment the variant in a detergent composition according to the invention is a polypeptide encoded by a polynucleotide having at least 90% identity to the mature polypeptide coding sequence of SEQ ID NO: 1 or 3 or a sequence encoding the mature polypeptide of SEQ ID NO: 2 or 4. In one embodiment the variant in a detergent composition according to the invention is a polypeptide encoded by a polynucleotide having least 90%, at least 91%, at least 92%, at least 93%, at least 94%at least 95% identity, at least 96%, at least 97%, at least 98%, or at least 99%, but less

than 100%, sequence identity to the mature polypeptide of SEQ ID NO: 1 or 3.

Another embodiment concerns a method for producing a detergent composition which comprises the step of adding a subtilisin variant which was obtained by a method comprising introducing into a parent subtilisin having at least 90%, at least 95%, at least 96%, at least 97%, at least 98%, at least 99% or 100% sequence identity to the mature polypeptide of SEQ ID NO: 2 or 4 the substitutions corresponding to Y217X and N218Z of SEQ ID NO: 2, wherein X and Z are selected from the group consisting of D or, E, and wherein the number of alterations in the subtilisin variant is 1-20, *e.g.*, 1-10 and 1-5, such as 1, 2, 3, 4, 5, 6, 7, 8, 9 or 10 alterations. Of course, preferably the variant has protease activity. A particular aspect concerns a method for producing a detergent composition which comprises the step of adding a subtilisin variant which was obtained by a method comprising introducing into a parent subtilisin the substitutions corresponding to Y217X and N218Z of SEQ ID NO: 2, wherein X and Z are selected from the group consisting of D or E, wherein the variant is a variant of a subtilisin parent protease selected from the group consisting of:

a. a polypeptide having at least 90% sequence identity to the mature polypeptide of SEQ ID NO: 2 or 4;

b. a polypeptide encoded by a polynucleotide having at least 90% identity to the mature polypeptide coding sequence of SEQ ID NO: 1 or 3, or the cDNA sequence thereof.

Another aspect of the invention relates to a detergent composition which was Obtained by a method as described above.

In one embodiment the protease variant in the detergent composition of the invention is a BPN' (SEQ ID NO: 2) variant comprising the substitutions corresponding to Y217X and N218Z of SEQ ID NO: 2, wherein X and Z are selected from the group consisting of D, E, the subtilisin variant having at least 90%, at least 95%, at least 96%, at least 97%, at least 98%, or at least 99%, but less than 100% sequence identity to the mature polypeptide of SEQ ID NO: 2, and wherein the number of alterations in the subtilisin variant is 1-20, *e.g.*, 1-10 and 1-5, such as 1, 2, 3, 4, 5, 6, 7, 8, 9 or 10 alterations.

. In yet another embodiment the protease variant in the detergent composition of the invention is a BPN' variant comprising any of the substitutions selected from the group consisting of [Y217D + N218D], [Y217E + N218E], [Y217D + N218E], [Y217E + N218D], at least 90%, at least 91%, at least 92%, at least 93%, at least 94%at least 95% identity, at least 96%, at least 97%, at least 98%, or at least 99%, but less than 100%, sequence identity to the mature polypeptide of SEQ ID NO: 2.

In another particular embodiment the protease variant in the detergent composition of the invention is a Savinase (SEQ ID NO: 4) variant comprising the substitutions corresponding to L217X and N218Z of SEQ ID NO: 2, wherein X and Z are selected from the group consisting of D, , the subtilisin variant having at least 90%, at least 95%, at least 96%, at least 97%, at least 98%, or at least 99%, but less than 100% sequence identity to the mature polypeptide of SEQ ID NO: 2, and wherein the number of alterations in the subtilisin variant is 1-20, *e.g.*, 1-10 and 1-5, such as 1, 2, 3, 4, 5, 6, 7, 8, 9 or 10 alterations. In another embodiment the protease variant in the detergent composition of the invention is a Savinase variant comprising any of the substitutions selected from the group consisting of [L217D + N218D], [L217E + N218E], [L217D + N218E], [L217E + N218D], least 94%at least 95% identity, at least 96%, at least 97%, at least 98%, or at least 99%, but less than 100%, sequence identity to the mature polypeptide of SEQ ID NO: 4.

In one embodiment the variant comprises a substitution at a position corresponding to position 217 of SEQ ID NO: 2 with D and further comprises a substitution at a position corresponding to position 218 of SEQ ID NO: 2 with D.

In one embodiment the variant comprises a substitution at a position corresponding to position 217 of SEQ ID NO: 2 with E and further comprises a substitution at a position corresponding to position 218 of SEQ ID NO: 2 with D.

In one embodiment the variant comprises a substitution at a position corresponding to position 217 of SEQ ID NO: 2 with D and further comprises a substitution at a position corresponding to position 218 of SEQ ID NO: 2 with E.

In one embodiment the variant comprises a substitution at a position corresponding to position 217 of SEQ ID NO: 2 with E and further comprises a substitution at a position corresponding to position 218 of SEQ ID NO: 2 with E.

In one aspect, the variant comprises or consists of an alteration at a position corresponding to position 217. In another aspect, the amino acid at a position corresponding to position 217 is substituted with Asp, Glu, preferably with Asp. In another aspect, the variant comprises or consists of the substitution Y217D of the mature polypeptide of SEQ ID NO: 2.

In one aspect, the variant comprises or consists of an alteration at a position corresponding to position 218. In another aspect, the amino acid at a position corresponding to position 218 is substituted with Asp, Glu.

In one aspect, the variant comprises or consists of an alteration at a position corresponding to position 217 and an alteration at a position corresponding position 218 wherein said position 217 and 218 are substituted with Asp, Glu. In another aspect, the variant comprises or consists of an alteration at a position corresponding to position 217 and an alteration at a position corresponding position 218 wherein said position 217 and 218 are substituted with Asp, Glu, and wherein the substitution at position 217 and 218 is with the same amino acid. Thus in one aspect the variant

comprises or consists of the substitutions Y217D + N218D, Y217E + N218E. In another aspect, the variant comprises or consists of an alteration at a position corresponding to position 217 and an alteration at a position corresponding position 218 of SEQ ID NO: 2 with Asp, Glu, wherein the substitution at position 217 and 218 is not with the same amino acid and wherein the substitution at position 217 and 218 result in an overall change of charge at these two positions. Thus in one aspect the variant comprises or consists of the substitutions Y217D + N218E, Y217E + N218D. All variants described above are particularly applicable in detergent compositions according to the invention.

Thus one aspect of the invention concerns a detergent composition comprising a subtilisin variant, comprising the substitutions corresponding to Y217X and N218Z of SEQ ID NO: 2, wherein X and Z are selected from the group consisting of D, E and wherein X and Z are the same amino acid.

One aspect of the invention concerns a detergent composition comprising a subtilisin variant, comprising the substitutions corresponding to Y217X and N218Z of SEQ ID NO: 2, wherein X and Z are selected from the group consisting of D, E, wherein X and Z are the same amino acid and wherein X and Z are D.

One aspect of the invention concerns a detergent composition comprising a subtilisin variant, comprising the substitutions corresponding to Y217X and N218Z of SEQ ID NO: 2, wherein X and Z are selected from the group consisting of D, E, wherein X and Z are the same amino acid and wherein X and Z are E.

One aspect of the invention concerns a detergent composition comprising a subtilisin variant, comprising the substitutions corresponding to Y217X and N218Z of SEQ ID NO: 2, wherein X and Z are selected from the group consisting of D, E, wherein X and Z are not the same amino acid and wherein X is D and Z is E.

One aspect of the invention concerns a detergent composition comprising a subtilisin variant, comprising the substitutions corresponding to Y217X and N218Z of SEQ ID NO: 2, wherein X and Z are selected from the group consisting of D, E, wherein X and Z are not the same amino acid and wherein X is E and Z is D.

In another aspect, the variant comprises or consists of the substitutions L217D and N218D of the mature polypeptide of SEQ ID NO: 4.

In another aspect, the variant comprises or consists of the substitutions L217E and N218E of the mature polypeptide of SEQ ID NO: 4.

In another aspect, the variant comprises or consists of the substitutions L217D and N218E of the mature polypeptide of SEQ ID NO: 4.

In another aspect, the variant comprises or consists of the substitutions L217E and N218D of the mature polypeptide of SEQ ID NO: 4.

The variants in detergent compositions according to the invention may further comprise one or more additional alterations at one or more (*e.g.*, several) other positions.

The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a polyhistidine tract, an antigenic epitope or a binding domain.

Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. Common substitutions are Ala/Ser, Val/Ile, Asp/Glu, Asn/Gln, Thr/Ser, Ala/Gly, Ala/Thr, Ser/Asn, Ala/Val, Ser/Gly, Tyr/Phe, Ala/Pro, Lys/Arg, Asp/Asn, Glu/Gln, Leu/Ile, Leu/Val, Ala/Glu, and Asp/Gly.

Alternatively, the amino acid changes are of such a nature that the physico-chemical properties of the polypeptides are altered. For example, amino acid changes may improve the thermal stability of the polypeptide, alter the substrate specificity, change the pH optimum, and the like.

For example, the variants in detergent compositions according to the invention may comprise an alteration at the positions corresponding to positions 217 and 218 and further comprise an alteration at another position(s) as long as the alteration does not affect the performance of the variant.

Essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for protease activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identity of essential amino acids can also be inferred from an alignment

with a related polypeptide. For BPN' (SEQ ID NO: 2) the catalytic triad comprising the amino acids S221, H64, and D32 is essential for protease activity of the enzyme.

The variants may consist of 200 to 900 amino acids, e.g., 210 to 800, 220 to 700, 230 to 600, 240 to 500, 250 to 400, 255 to 300, 260 to 290, 265 to 285, 270 to 280 or 270, 271, 272, 273, 274, 275, 276, 277, 278, 279 or 280 amino acids.

In an embodiment, the variant has improved catalytic activity compared to the parent enzyme.

In an embodiment, the variant has improved wash performance compared to the parent subtilisin or compared to a protease having the identical amino acid sequence of said variant but not having the alterations at one or more of said specified positions or compared to a protease with SEQ ID NO: 2, wherein the wash performance is measured as described in example 2 in "Material and Methods" herein.

In another embodiment, the detergent composition comprising the variant has an improved wash performance compared to an otherwise identical detergent composition with a protease being the parent subtilisin or with a protease having the identical amino acid sequence of said variant but not having the alterations at one or more of said specified positions, or with a protease with SEQ ID NO: 2.

**Parent proteases**

Enzymes cleaving the amide linkages in protein substrates are classified as proteases, or (interchangeably) peptidases (see Walsh, 1979, Enzymatic Reaction Mechanisms. W.H. Freeman and Company, San Francisco, Chapter 3).

Serine proteases

A serine protease is an enzyme which catalyzes the hydrolysis of peptide bonds, and in which there is an essential serine residue at the active site (White, Handler and Smith, 1973 "Principles of Biochemistry," Fifth Edition, McGraw-Hill Book Company, NY, pp. 271-272).

The bacterial serine proteases have molecular weights in the 20,000 to 45,000 Dalton range. They are inhibited by diisopropylfluorophosphate. They hydrolyze simple terminal esters and are similar in activity to eukaryotic chymotrypsin, also a serine protease. A more narrow term, alkaline protease, covering a sub-group, reflects the high pH optimum of some of the serine proteases, from pH 9.0 to 11.0 (for review, see Priest (1977) Bacteriological Rev. 41 711-753).

Subtilases

A sub-group of the serine proteases tentatively designated subtilases has been proposed by Siezen et al., Protein Engng. 4 (1991) 719-737 and Siezen et al. Protein Science 6 (1997) 501-523. They are defined by homology analysis of more than 170 amino acid sequences of serine proteases previously referred to as subtilisin-like proteases. A subtilisin was previously often defined as a serine protease produced by Gram-positive bacteria or fungi, and according to Siezen et al. now is a subgroup of the subtilases. A wide variety of subtilases have been identified, and the amino acid sequence of a number of subtilases has, been determined. For a more detailed description of such subtilases and their amino acid sequences reference is made to Siezen et al. (1997).

One subgroup of the subtilases, I-S1 or "true" subtilisins, comprises the "classical" subtilisins, such as subtilisin 168 (BSS168), subtilisin BPN', subtilisin Carlsberg (ALCALASE®, NOVOZYMES A/S), and subtilisin DY (BSSDY).

A further subgroup of the subtilases, I-S2 or high alkaline subtilisins, is recognized by Siezen et al. (supra). Sub-group I-S2 proteases are described as highly alkaline subtilisins and comprises enzymes such as subtilisin PB92 (BAALKP) (MAXACAL®, Genencor International Inc.), subtilisin 309 (SAVINASE®, NOVOZYMES A/S), subtilisin 147 (BLS147) (ESPERASE®, NOVOZYMES A/S), and alkaline elastase YaB. (BSEYAB). BPN' is subtilisin BPN' from B. amyloliquefaciens BPN' has the amino acid sequence SEQ ID NO: 2.

Subtilisins

Subtilisins are serine proteases from the family S8, in particular from the subfamily S8A, as defined by the MEROPS database (http://merops.sanger.ac.uk/cgi-bin/famsum?family=S8).
BPN' and Savinase have the MEROPS numbers S08.034 and S08.003, respectively.

**Parent subtilisin**

The term "parent subtilisin" describes a subtilase defined according to Siezen et al. (1991 and 1997). For further details see description of "Subtilases" above. A parent subtilisin may also be a subtilase isolated from a natural source,

wherein subsequent modifications have been made while retaining the characteristic of a subtilase. Furthermore, a parent subtilisin may be a subtilase which has been prepared by the DNA shuffling technique, such as described by J.E. Ness et al., Nature Biotechnology, 17, 893-896 (1999).

Alternatively the term "parent subtilisin" may be termed "wild type subtilase".

For reference a table of the acronyms for various subtilases mentioned herein is provided, for further acronyms, see Siezen et al., Protein Engng: 4 (1991) 719-737 and Siezen et al. Protein Science 6 (1997) 501-523.

**Table III**

| Organism | enzyme | acronym |
|---|---|---|
| **Bacteria: Gram-positive** | | |
| *Bacillus subtilis* 168 | subtilisin I168,apr | BSS168 |
| *Bacillus amyloliquefaciens* | subtilisin BPN' (NOVO) | BASBPN |
| *Bacillus subtilis DY* | subtilisin DY | BSSDY |
| *Bacillus licheniformis* | subtilisin Carlsberg | BLSCAR |
| *Bacillus lentus* | subtilisin 309 | BLSAVI |
| *Bacillus lentus* | subtilisin 147 | BLS147 |
| *Bacillus alcalophilus* PB92 | subtilisin PB92 | BAPB92 |
| *Bacillus* YaB | alkaline elastase YaB | BYSYAB |
| *Bacillus sp.* NKS-21 | subtilisin ALP I | BSAPRQ |
| *Bacillus sp.* G-825-6 | subtilisin Sendai | BSAPRS |
| *Thermoactinomyces vulgaris* | thermitase | TVTHER |

Modification(s) of a subtilase

The term "modification(s)" used herein is defined to include chemical modification of a subtilase as well as genetic manipulation of the DNA encoding a subtilase. The modification(s) can be replacement(s) of the amino acid side chain(s), substitution(s), deletion(s) and/or insertion(s) in or at the amino acid(s) of interest.

Subtilisin variant

The term "variant" and the term "subtilisin variant" are defined above.

Homologous subtilase sequences

The homology between two amino acid sequences is in this context described by the parameter "identity" for purposes of the present invention, the degree of identity between two amino acid sequences is determined using the Needleman-Wunsch algorithm as described above. The output from the routine is besides the amino acid alignment the calculation of the "Percent Identity" between the two sequences.

Based on this description it is routine for a person skilled in the art to identify suitable homologous subtilases, which can be modified according to the invention

Substantially homologous parent subtilisin variants may have one or more (several) amino acid substitutions, deletions and/or insertions, in the present context the term "one or more" is used interchangeably with the term "several". These changes are preferably of a minor nature, that is conservative amino acid substitutions as described above and other substitutions that do not significantly affect the three-dimensional folding or activity of the: protein or polypeptide; small deletions, typically of one to about 30 amino acids; and small amino- or carboxyl-terminal extensions, such as an amino-terminal methionine residue, a small linker peptide of up to about 20-25 residues, or a small extension that facilitates purification (an affinity tag), such as a poly-histidine tract, or protein A (Nilsson et al., 1985, EMBO J. 4: 1075; Nilsson et al., 1991, Methods Enzymol. 198: 3. See, also, in general, Ford et al., 1991, Protein Expression and Purification 2: 95-107.

Although the changes described above preferably are of a minor nature, such changes may also be of a substantive nature such as fusion of larger polypeptides of up to 300 amino acids or more both as amino- or carboxyl-terminal extensions.

The parent subtilisin may comprise or consist of the amino acid sequence of SEQ ID NO: 2 or an allelic variant thereof. In one aspect, the parent subtilisin comprises or Consists of the amino acid sequence of SEQ ID NO: 2.

The parent subtilisin may be (a) a polypeptide having at least 90% sequence identity to the mature polypeptide of SEQ ID NO: 2 or 4; or (b) a polypeptide encoded by a polynucleotide having at least 960% sequence identity to the

mature polypeptide coding sequence of SEQ ID NO: 1 or 3.

In an aspect, the parent has a sequence identity to the mature polypeptide of SEQ ID NO: 2 or 4 of at least 90%, at least 91%, at least 92%, at least 93%, at least 94%at least 95% identity, at least 96%, at least 97%, at least 98%, or at least 99%, or 100%, which have protease activity In one aspect, the amino acid sequence of the parent differs by no more than 10 amino acids, *e.g.,* 1, 2, 3, 4, 5, 6, 7, 8, or 9, from the mature polypeptide of SEQ ID NO: 2 or 4. In one preferred aspect, the amino acid sequence of the parent differs by no more than 10 amino acids, *e.g.,* 1, 2, 3, 4, 5, 6, 7, 8, or 9, from the mature polypeptide of SEQ ID NO: 2 or 4 wherein the variants have retained its improved properties.

In another aspect, the parent comprises or consists of the amino acid sequence of SEQ ID NO: 2 or 4. In another aspect, the parent comprises or consists of the mature polypeptide of SEQ ID NO: 2 or 4 In another aspect, the parent comprises or consists of amino acids 1 to 275 of SEQ ID NO: 2. In yet another aspect, the parent comprises or consists of amino acids 1 to 269 of SEQ ID NO: 4.

A genomic DNA or cDNA library prepared from such other strains may be screened for DNA that hybridizes with the probes described above and encodes a parent. Genomic or other DNA from such other strains may be separated by agarose or polyacrylamide gel electrophoresis, or other separation techniques. DNA from the libraries or the separated DNA may be transferred to and immobilized on nitrocellulose or other suitable carrier material. In order to identify a clone or DNA that hybridizes with SEQ ID NO: 1 or 3 or a subsequence thereof, the carrier material is used in a Southern blot.

For purposes of the present invention, hybridization indicates that the polynucleotide hybridizes to a labeled nucleic acid probe corresponding to (i) SEQ ID NO: 1 or 3; (ii) the mature polypeptide coding sequence of SEQ ID NO: 1 or 3; (iii) a sequence encoding the mature polypeptide of SEQ ID NO: 2 or 4; (iv) the full-length complement thereof; or (v) a subsequence thereof; under very low to very high stringency conditions. Molecules to which the nucleic acid probe hybridizes under these conditions can be detected using, for example, X-ray film or any other detection means known in the art.

In one aspect, the nucleic acid probe is the mature polypeptide coding sequence of SEQ ID NO: 1 or 3. In another aspect, the nucleotide acid probe is a 80 to 1140 nucleotides long fragment of SEQ ID NO: 1 or 3, e.g. 90, 100, 200, 300, 400, 500, 600, 700, 800, 900, 1000 or 1100 nucleotides long. In another aspect, the nucleic acid probe is a polynucleotide that encodes the polypeptide of SEQ ID NO: 2 or 4; the mature polypeptide thereof; or a fragment thereof. In another aspect, the nucleic acid probe is SEQ ID NO: 1 or 3 or a sequence encoding the mature polypeptide of SEQ ID NO: 2 or 4 respectively.

In another embodiment, the parent is encoded by a polynucleotide having a sequence identity to the mature polynucleotide coding sequence of SEQ ID NO: 1 or 3 of at least 90%, at least 91%, at least 92%, at least 93%, at least 94%at least 95% identity, at least 96%, at least 97%, at least 98%, or at least 99% or 100%.

The polypeptide may be a hybrid polypeptide in which a region of one polypeptide is fused at the N-terminus or the C-terminus of a region of another polypeptide.

The parent may be a fusion polypeptide or cleavable fusion polypeptide in which another polypeptide is fused at the N-terminus or the C-terminus of the polypeptide of the present invention. A fusion polypeptide is produced by fusing a polynucleotide encoding another polypeptide to a polynucleotide of the present invention. Techniques for producing fusion polypeptides are known in the art, and include ligating the coding sequences encoding the polypeptides so that they are in frame and that expression of the fusion polypeptide is under control of the same promoter(s) and terminator. Fusion polypeptides may also be constructed using intein technology in which fusion polypeptides are created post-translationally (Cooper et al., 1993, EMBO J. 12: 2575-2583; Dawson et al., 1994, Science 266: 776-779).

A fusion polypeptide can further comprise a cleavage site between the two polypeptides. Upon secretion of the fusion protein, the site is cleaved releasing the two polypeptides. Examples of cleavage sites include, but are not limited to, the sites disclosed in Martin et al., 2003, J. Ind. Microbiol. Biotechnol. 3: 568-576; Svetina et al., 2000, J. Biotechnol. 76: 245-251; Rasmussen-Wilson et al., 1997, Appl. Environ. Microbiol. 63: 3488-3493; Ward et al., 1995, Biotechnology 13: 498-503; and Contreras et al., 1991, Biotechnology 9: 378-381; Eaton et al., 1986, Biochemistry 25: 505-512; Collins-Racie et al., 1995, Biotechnology 13: 982-987; Carter et al., 1989, Proteins: Structure, Function, and Genetics 6: 240-248; and Stevens, 2003, Drug Discovery World 4: 35-48.

The parent may be obtained from organisms of any genus. For purposes of the present invention, the term "obtained from" as used herein in connection with a given source shall mean that the parent encoded by a polynucleotide is produced by the source or by a strain in which the polynucleotide from the source has been inserted. In one aspect, the parent is secreted extracellularly.

The parent may be a bacterial protease. For example, the parent may be a Gram-positive bacterial polypeptide such as a *Bacillus, Clostridium, Enterococcus, Geobacillus, Lactobacillus, Lactococcus, Oceanobacillus, Staphylococcus, Streptococcus,* or *Streptomyces* protease, or a Gram-negative bacterial polypeptide such as a *Campylobacter, E. coli, Flavobacterium, Fusobacterium, Helicobacter, Ilyobacter, Neisseria, Pseudomonas, Salmonella,* or *Ureaplasma* protease.

In one aspect, the parent is a *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus meg-*

*aterium, Bacillus pumilus, Bacillus stearothermophilus, Bacillus subtilis,* or *Bacillus thuringiensis* protease

In one aspect, the parent is a *Bacillus amyloliquefaciens* protease, *e.g.,* the protease of SEQ ID NO: 2 or the mature polypeptide thereof.

In another aspect, the parent is a *Bacillus lentus* protease, *e.g.,* the protease of SEQ ID NO: 4 or the mature polypeptide thereof.

Strains of these species are readily accessible to the public in a number of culture collections, such as the American Type Culture Collection (ATCC), Deutsche Sammlung von Mikroorganismen und Zellkulturen GmbH (DSMZ), Centraalbureau Voor Schimmelcultures (CBS), and Agricultural Research Service Patent Culture Collection, Northern Regional Research Center (NRRL).

The parent may be identified and obtained from other sources including microorganisms isolated from nature (e.g., soil, composts, water, etc.) or DNA samples obtained directly from natural materials (e.g., soil, composts, water, etc.) using the above-mentioned probes. Techniques for isolating microorganisms and DNA directly from natural habitats are well known in the art. A polynucleotide encoding a parent may then be obtained by similarly screening a genomic DNA or cDNA library of another microorganism or mixed DNA sample. Once a polynucleotide encoding a parent has been detected with the probe(s), the polynucleotide can be isolated or cloned by utilizing techniques that are known to those of ordinary skill in the art (see, *e.g.,* Sambrook *et al.,* 1989, *supra*).

**Preparation of Variants**

The variants can be prepared using any mutagenesis procedure known in the art, such as site-directed mutagenesis, synthetic gene construction, semi-synthetic gene construction, random mutagenesis, shuffling, etc.

Site-directed mutagenesis is a technique in which one or more (*e.g.*, several) mutations are introduced at one or more defined sites in a polynucleotide encoding the parent.

Site-directed mutagenesis can be accomplished *in vitro* by PCR involving the use of oligonucleotide primers containing the desired mutation. Site-directed mutagenesis can also be performed *in vitro* by cassette mutagenesis involving the cleavage by a restriction enzyme at a site in the plasmid comprising a polynucleotide encoding the parent and subsequent ligation of an oligonucleotide containing the mutation in the polynucleotide. Usually the restriction enzyme that digests the plasmid and the oligonucleotide is the same, permitting sticky ends of the plasmid and the insert to ligate to one another. See, e.g., Scherer and Davis, 1979, Proc. Natl. Acad. Sci. USA 76: 4949-4955; and Barton et al., 1990, Nucleic Acids Res. 18: 7349-4966.

Site-directed mutagenesis can also be accomplished *in vivo* by methods known in the art. See, e.g., U.S. Patent Application Publication No. 2004/0171154; Storici et al., 2001, Nature Biotechnol. 19: 773-776; Kren et al., 1998, Nat. Med. 4: 285-290; and Calissano and Macino, 1996, Fungal Genet. Newslett. 43: 15-16.

Any site-directed mutagenesis procedure can be used in the present invention. There are many commercial kits available that can be used to prepare variants.

Synthetic gene construction entails *in vitro* synthesis of a designed polynucleotide molecule to encode a polypeptide of interest. Gene synthesis can be performed utilizing a number of techniques, such as the multiplex microchip-based technology described by Tian et al. (2004, Nature 432: 1050-1054) and similar technologies wherein oligonucleotides are synthesized and assembled upon photo-programmable microfluidic chips.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (e.g., Lowman et al., 1991, Biochemistry 30: 10832-10837; U.S. Patent No. 5,223,409; WO 92/06204) and region-directed mutagenesis (Derbyshire et al., 1986, Gene 46: 145; Ner et al., 1988, DNA 7: 127).

Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

Semi-synthetic gene construction is accomplished by combining aspects of synthetic gene construction, and/or site-directed mutagenesis, and/or random mutagenesis, and/or shuffling. Semi-synthetic construction is typified by a process utilizing polynucleotide fragments that are synthesized, in combination with PCR techniques. Defined regions of genes may thus be synthesized de *novo,* while other regions may be amplified using site-specific mutagenic primers, while yet other regions may be subjected to error-prone PCR or non-error prone PCR amplification. Polynucleotide subsequences may then be shuffled.

**Further characterization of detergent compositions according to the invention**

According to the invention, it has surprisingly been found that detergent compositions as described above, i.e. detergent compositions comprising a protease variant as described, exhibit an advantageous wash performance, preferably an improved wash performance, especially with regard to protease-sensitive stains, particularly egg stains.
The detergent composition of the present invention may be formulated, for example, as a hand or machine laundry detergent composition including a laundry additive composition suitable for pre-treatment of stained fabrics and a rinse added fabric softener composition, or be formulated as a detergent composition for use in general household hard surface cleaning operations, or be formulated for hand or machine dishwashing operations. Preferably, the detergent composition according to the invention is a laundry detergent composition or a dishwashing composition, preferably a machine dishwashing composition.
In a specific aspect, the present invention provides a detergent additive comprising a polypeptide of the present invention as described herein.
In a further preferred embodiment, the detergent composition according to the invention comprises the protease variant in an amount from 1 x $10^{-8}$-10 weight (wt.) percent, from 0,00001-2 wt.%, from 0,001-1 wt.%, from 0,007-0,8 wt.%, from 0,025-0,5 Gew.-% and particularly preferred from 0,04-0,38 wt.-%, based on total protein content of the protease variant. The protein concentration can be determined using known methods, for example the BCA Process (bicinchoninic acid; 2,2'-biquinolyl-4,4'-dicarboxylic acid) or the biuret process (A. G. Gornall, C. S. Bardawill and M.M. David, J. Biol. Chem., 177 (1948), pp. 751-766).
In another preferred embodiment, the detergent composition according to the invention is a liquid detergent composition.
In a further preferred embodiment, the detergent composition is characterized in that it exhibits an improved wash performance. Preferably, said improved wash performance is determined as indicated in the definitions herein, especially by calculating the so-called intensity value (Int) defined in AMSA assay as described in Materials and methods herein (see also the wash performance test in Example 2 herein) or by the reference washing test described afterwards.

Reference washing test:

In the reference washing test, the wash performance is determined in a washing system that contains a detergent composition at a dosing ratio of between 4.5 and 7.0 grams per liter of washing liquor as well as the protease. The detergent compositions to be compared contain the same amount of the respective protease based on total protein content, preferably providing for an amount of 5 mg protease per liter wash liquor. The wash performance is determined with respect to egg stains, in particular with respect to one or more of the following stains:

- full egg with pigment on cotton: product no. C-S-37 obtainable from CFT (Center for Testmaterials) B.V., Vlaardingen, Netherlands,
- full egg with pigment on cotton/polyester: product no. PC-S-37 obtainable from CFT (Center for Testmaterials) B.V., Vlaardingen, Netherlands,
- full egg with pigment, aged by heating, on cotton/polyester: product no. PC-S-39 obtainable from CFT (Center for Testmaterials) B.V., Vlaardingen, Netherlands,

by measuring the whiteness of the washed textiles, the washing procedure being performed for at least 30 minutes, optionally 60 minutes, at a temperature of 40°C, and the water having a water hardness between 15.5 and 16.5° (German degrees of hardness).
The preferred liquid detergent composition for said washing system has the following composition (all indications in percentage by weight): 0.3 to 0.5% xanthan gum, 0.2 to 0.4% antifoaming agent, 6 to 7% glycerol, 0.3 to 0.5% ethanol, 4 to 7% FAEOS (fatty alcohol ether sulfate), 24 to 28% nonionic surfactants, 1% boric acid, 1 to 2% sodium citrate (dihydrate), 2 to 4% soda, 14 to 16% coconut fatty acid, 0.5% HEDP (1-hydroxyethane-(1,1-diphosphonic acid)), 0 to 0.4% PVP (polyvinylpyrrolidone), 0 to 0.05% optical brighteners, 0 to 0.001% dye, remainder deionized water. The dosing ratio of the liquid washing agent is preferably between 4.5 and 6.0 grams per liter of washing liquor, for example 4.7, 4.9, or 5.9 grams per liter of washing liquor. Washing preferably occurs in a pH range between pH 8 and pH 10.5, preferably between pH 8 and pH 9.
The preferred powdered detergent composition for said washing system has the following composition (all indications in percentage by weight): 10% linear alkylbenzenesulfonate (sodium salt), 1.5% C12 to C18 fatty alcohol sulfate (sodium salt), 2.0% C12 to C18 fatty alcohol with 7 EO, 20% sodium carbonate, 6.5% sodium hydrogencarbonate, 4.0% amorphous sodium disilicate, 17% sodium carbonate peroxohydrate, 4.0% TAED, 3.0% polyacrylate, 1.0% carboxymethyl cellulose, 1.0% phosphonate, 25% sodium sulfate; remainder: optionally foam inhibitors, optical brighteners, scents, and if applicable water to make 100%. The dosing ratio of the powdered washing agent is preferably between

5.5 and 7.0 grams per liter of washing liquor, for example 5.6, 5.9, or 6.7 grams per liter of washing liquor. Washing preferably occurs in a pH range between pH 9 and pH 11.

It is preferred according to the present invention if the aforementioned liquid washing agent is used, as indicated, to determine the wash performance by the reference washing test.

The whiteness, i.e. the brightening of the stains, is determined as an indication of wash performance, preferably using optical measurement methods, preferably photometrically. A device suitable for this is, for example, the Minolta CM508d spectrometer. The devices used for measurement are usually calibrated beforehand using a white standard, preferably a white standard provided with the unit.

The enzyme(s) of the detergent compositions of the invention, especially the protease variants described herein, may be stabilized using conventional stabilizing agents, e.g., a polyol such as propylene glycol or glycerol, a sugar or sugar alcohol, lactic acid, boric acid, or a boric acid derivative, e.g., an aromatic borate ester, or a phenyl boronic acid derivative such as 4-formylphenyl boronic acid, and the composition may be formulated as described in, for example, WO92/19709 and WO92/19708 or the variants according to the invention may be stabilized using peptide aldehydes or ketones such as described in WO2005/105826 and WO2009/118375.

A detergent composition according to the invention might further comprise one or more peroxy compounds. Such peroxy compounds optionally present in the compositions which may in particular be considered are organic peracids or peracidic salts of organic acids, such as phthalimidopercaproic acid, perbenzoic acid or salts of diperdodecanedioic acid, hydrogen peroxide and inorganic salts which release hydrogen peroxide under the washing conditions, such as perborate, percarbonate and/or persilicate. Hydrogen peroxide may here also be produced with the assistance of an enzymatic system, i.e. an oxidase and its substrate. Where solid peroxy compounds are to be used, they may be used in the form of powders or granules, which may also in principle be encapsulated in known manner. Alkali metal percarbonate, alkali metal perborate monohydrate, alkali metal perborate tetrahydrate or hydrogen peroxide in the form of aqueous solutions containing 3 wt.% to 10 wt.% of hydrogen peroxide are particularly preferred. Peroxy compounds are preferably present in washing or cleaning agents according to the invention in quantities of up to 50 wt.%, in particular of 5 wt.% to 30 wt.%.

Apart from the protease variant to be used according to the invention, the detergent compositions according to the invention, which may in particular take the form of pulverulent solids, post-compressed particles, homogeneous solutions or suspensions, may in principle contain any ingredients known and conventional in such compositions. The compositions according to the invention may in particular contain builder substances, surface-active surfactants, water-miscible organic solvents, enzymes, sequestering agents, electrolytes, pH regulators, polymers with specific effects, such as soil release polymers, dye transfer inhibitors, graying inhibitors, crease-reducing polymeric active ingredients and shape-retaining polymeric active ingredients, and further auxiliary substances, such as optical brighteners, foam regulators, additional peroxy activators, colorants and scents.

In addition to the above-stated ingredients, a composition according to the invention may contain conventional antimicrobial active ingredients in order to enhance the disinfection action, for example towards specific microorganisms. Such antimicrobial additives are preferably present in the disinfectants according to the invention in quantities of up to 10 wt.%, in particular of 0.1 wt.% to 5 wt.%.

In a detergent composition according to the invention, conventional bleach activators which form peroxycarboxylic acid or peroxyimidic acids under perhydrolysis conditions and/or conventional bleach-activating transition metal complexes may also be used. The bleach activator component optionally present in particular in quantities of 0.5 wt.% to 6 wt.% comprises conventionally used Nor O-acyl compounds, for example polyacylated alkylenediamines, in particular tetraacetylethylenediamine, acylated glycolurils, in particular tetraacetylglycoluril, N-acylated hydantoins, hydrazides, triazoles, urazoles, diketopiperazines, sulfurylamides and cyanurates, moreover carboxylic anhydrides, in particular phthalic anhydride, carboxylic acid esters, in particular sodium isononanoylphenolsulfonate, and acylated sugar derivatives, in particular pentaacetyl glucose, together with cationic nitrile derivatives such as trimethylammonium acetonitrile salts. In order to avoid interaction with per compounds during storage, the bleach activators may in known manner have been coated with shell substances or granulated, particularly preferred options being tetraacetylethylenediamine granulated with the assistance of carboxymethylcellulose and having an average grain size of 0.01 mm to 0.8 mm, granulated 1,5-diacetyl-2,4-dioxohexahydro-1,3,5-triazine, and/or trialkylammonium acetonitrile formulated in particulate form. Such bleach activators are preferably present in washing or cleaning compositions in quantities of up to 8 wt.%, in particular of 2wt.% to 6wt.%, in each case relative to entire composition.

The compositions according to the invention may contain one or more surfactants, with anionic surfactants, nonionic surfactants and mixtures thereof in particular being considered, but cationic and/or amphoteric surfactants also possibly being present. Suitable nonionic surfactants are in particular alkyl glycosides and ethoxylation and/or propoxylation products of alkyl glycosides or linear or branched alcohols in each case having 12 to 18 C atoms in the alkyl moiety and 3 to 20, preferably 4 to 10, alkyl ether groups. Corresponding ethoxylation and/or propoxylation products of N-alkylamines, vicinal diols, fatty acid esters and fatty acid amides, which correspond with regard to the alkyl moiety to the stated long-chain alcohol derivatives, and of alkylphenols having 5 to 12 C atoms in the alkyl residue may furthermore

be used.

Suitable anionic surfactants are in particular soaps and those which contain sulfate or sulfonate groups with preferably alkali metal ions as cations. Usable soaps are preferably the alkali metal salts of saturated or unsaturated fatty acids with 12 to 18 C atoms. Such fatty acids may also be used in incompletely neutralized form. Usable surfactants of the sulfate type include the salts of sulfuric acid semiesters of fatty alcohols with 12 to 18 C atoms and the sulfation products of the stated nonionic surfactants with a low degree of ethoxylation. Usable surfactants of the sulfonate type include linear alkylbenzene sulfonates with 9 to 14 C atoms in the alkyl moiety, alkane-sulfonates with 12 to 18 C atoms, and olefin sulfonates with 12 to 18 C atoms, which arise from the reaction of corresponding monoolefins with sulfur trioxide, and alpha-sulfofatty acid esters which arise from the sulfonation of fatty acid methyl or ethyl esters.

Such surfactants are present in the washing compositions according to the invention in proportions of preferably 5 wt.% to 50 wt.%, in particular of 8 wt.% to 30 wt.%, while the disinfectants according to the invention and the cleaning compositions according to the invention preferably contain 0.1 wt.% to 20 wt.%, in particular 0.2 wt.% to 5 wt.% of surfactants.

The compositions according to the invention, in particular if they are compositions intended for treating textiles, may in particular contain one or more of the cationic, textile-softening substances of the general formulae X, XI, or XII as cationic active substances with a textile-softening action:

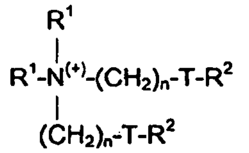

(X)

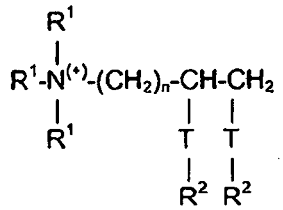

(XI)

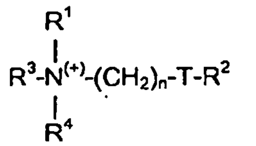

(XII)

in which each group $R^1$ is mutually independently selected from among $C_{1-6}$ alkyl, alkenyl or hydroxyalkyl groups; each group $R^2$ is mutually independently selected from among $C_{8-28}$ alkyl or alkenyl groups; $R^3 = R^1$ or $(CH_2)_n$-T-$R^2$; $R^4 = R^1$ or $R^2$ or $(CH_2)_n$-T-$R^2$; T = $-CH_2-$, -O-CO- or -CO-O- and n is an integer from 0 to 5. The cationic surfactants comprise conventional anions of a nature and number required for charge balancing, it being possible to select said anions not only from, for example, halides but also from anionic surfactants. In preferred embodiments of the present invention, cationic surfactants which may be used are hydroxyalkyltrialkylammonium compounds, in particular $C_{12-18}$ alkyl(hydroxyethyl)dimethylammonium compounds, and preferably the halides thereof, in particular chlorides. A composition according to the invention preferably contains 0.5 wt.% to 25 wt.%, in particular 1 wt.% to 15 wt.% of cationic surfactant.

A composition according to the invention preferably contains at least one water-soluble and/or water-insoluble, organic and/or inorganic builder. Water-soluble organic builder substances include polycarboxylic acids, in particular citric acid and saccharic acids, monomeric and polymeric aminopolycarboxylic acids, in particular methylglycinediacetic acid, nutrilotriacetic acid and ethylenediaminetetraacetic acid together with polyaspartic acid, polyphosphonic acids, in particular aminotris(methylenephosphonic acid), ethylenediamine-tetrakis(methylenephosphonic acid) and 1-hydroxyethane-1,1-diphosphonic acid, polymeric hydroxyl compounds such as dextrin and polymeric (poly-)carboxylic acids, in particular polycarboxylates obtainable by oxidizing polysaccharides or dextrins, and/or polymeric acrylic acids, methacrylic acids, maleic acids and copolymers thereof, which may also contain small proportions of polymerizable substances without carboxylic acid functionality. The relative molecular mass of the homopolymers of unsaturated carboxylic acids is in general between 5,000 and 200,000, that of the copolymers between 2,000 and 200,000, preferably 50,000 to 120,000, in each case relative to free acid. One particularly preferred acrylic acid/maleic acid copolymer has a relative molecular mass of 50.000 to 100,000. Suitable, albeit less preferred, compounds of this class are copolymers of acrylic acid or methacrylic acid with vinyl ethers, such as vinyl methyl ethers, vinyl esters, ethylene, propylene and styrene, the

acid fraction of which amounts to at least 50 wt.%. Terpolymers containing as monomers two unsaturated acids and/or the salts thereof and as third monomer, vinyl alcohol and/or an esterified vinyl alcohol or a carbohydrate may also be used as water-soluble organic builder substances. The first acidic monomer or the salt thereof is derived from a monoethylenically unsaturated $C_3$-$C_8$ carboxylic acid and preferably from a $C_3$-$C_4$ monocarboxylic acid, in particular from (meth)acrylic acid. The second acidic monomer or the salt thereof may be a derivative of a $C_4$-$C_8$ dicarboxylic acid, maleic acid being particularly preferred, and/or a derivative of an allylsulfonic acid which is substituted in position 2 with an alkyl or aryl residue. Such polymers generally have a relative molecular mass of between 1,000 and 200,000. Further preferred copolymers are those which comprise acrolein and acrylic acid/acrylic acid salts or vinyl acetate as monomers. The organic builder substances may be used, in particular for producing liquid compositions, in the form of aqueous solutions, preferably in the form of 30 to 50 wt.% aqueous solutions. All the stated acids are generally used in the form of the water-soluble salts, in particular the alkali metal salts, thereof.

Such organic builder substances may, if desired, be present in quantities of up to 40 wit.%, in particular of up to 25 wt.% and preferably of 1 wt.% to 8 wt.%. Quantities close to the stated upper limit are preferably used in pasty or liquid, in particular water-containing, compositions according to the invention.

Water-soluble inorganic builder materials which may in particular be considered are polymeric alkali metal phosphates, which may be present in the form of the alkaline, neutral or acidic sodium or potassium salts thereof. Examples are tetrasodium diphosphate, disodium dihydrogen diphosphate, pentasodium triphosphate, "sodium hexametaphosphate" and the corresponding potassium salts or mixtures of sodium and potassium salts. Water-insoluble, water-dispersible inorganic builder materials which are used are in particular crystalline or amorphous alkali metal aluminosilicates, in quantities of up to 50 wt.%, preferably of no more than 40 wt.% and, in liquid compositions, in particular from 1 wt.% to 5 wt.%. Among these, washing composition grade crystalline sodium aluminosilicates, in particular zeolite A, P and optionally X, are preferred. Quantities close to the stated upper limit are preferably used in solid, particulate compositions. Suitable aluminosilicates in particular comprise no particles with a grain size of above 30 $\mu$m and preferably consist to an extent of at least 80 wt.% of particles with a size below 10 $\mu$m Their calcium binding capacity, which may be determined as stated in German patent DE 24 12 837, is generally in the range of from 100 to 200 mg of CaO per gram.

Suitable substitutes or partial substitutes for the stated aluminosilicate are crystalline alkali metal silicates, which may be present alone or mixed with amorphous silicates. The alkali metal silicates usable as builders in the compositions according to the invention preferably have a molar ratio of alkali metal oxide to $SiO_2$ of below 0.95, in particular of 1:1.1 to 1:12 and may be in amorphous or crystalline form. Preferred alkali metal silicates are sodium silicates, in particular amorphous sodium silicates, with a molar ratio $Na_2O$:$SiO_2$ of 1:2 to 1:2.8. Preferably used crystalline silicates, which may be present alone or mixed with amorphous silicates, are crystalline phyllosilicates of the general formula $Na_2Si_xO_{2x+i} \cdot y\ H_2O$, in which x, the "modulus", is a number from 1.9 to 4 and y is a number from 0 to 20 and preferred values for x are 2, 3 or 4. Preferred crystalline phyllosilicates are those in which x in the stated general formula assumes the values 2 or 3. In particular, both $\beta$- and $\delta$-sodium disilicates ($Na_2Si_2O_5 \cdot y\ H_2O$) are preferred. Virtually anhydrous crystalline alkali metal silicates, produced from amorphous alkali metal silicates, of the above-stated general formula in which x means a number from 1.9 to 2.1 may also be used in compositions according to the invention. A crystalline sodium phyllosilicate with a modulus of 2 to 3, as may be produced from sand and soda, is used in a further preferred embodiment of compositions according to the invention. Crystalline sodium silicates with a modulus in the range from 1.9 to 3.5 are used in a further preferred embodiment of compositions according to the invention. In one preferred development of compositions according to the invention, a granular compound of alkali metal silicate and alkali metal carbonate is used, as is for example commercially available under the name Nabion® 15. If alkali metal aluminosilicate, in particular zeolite, is present as an additional builder substance, the weight ratio of aluminosilicate to silicate in each case relative to anhydrous active substances, preferably amounts to 1:10 to 10:1. In compositions which contain both amorphous and crystalline alkali metal silicates, the weight ratio of amorphous alkali metal silicate to crystalline alkali metal silicate preferably amounts to 1:2 to 2:1 and in particular to 1:1 to 2:1.

Builder substances are present in the washing or cleaning compositions according to the invention preferably in quantities of up to 60 wt.%, in particular of 5 wt.% to 40 wt.%.

In a preferred development of the invention, a composition according to the invention comprises a water-soluble "builder block". Use of the term "builder block" is intended to indicate that the composition does not contain any further builder substances as such which are water-soluble, i.e. all the builder substances present in the composition are combined in the "block" characterized in this manner, an exception being, however, made for the quantities of substances which may be present, as is conventional in commerce, in small quantities as contaminants or stabilizing additives in the other ingredients of the compositions. The term "water-soluble" should here be taken to mean that, at the concentration which is obtained under conventional conditions by the input quantity of the composition containing it, the builder block dissolves without leaving a residue. The compositions according to the invention preferably contain at least 15 wt.% and up to 55 wt,%, in particular 25 wt.% to 50 wt.% of water-soluble builder block. The latter is preferably composed of the components

a) 5 wt.% to 35 wt.% citric acid, alkali metal citrate and/or alkali metal carbonate, which may also at least in part be replaced by alkali metal hydrogencarbonate,
b) up to 10 wt.% alkali metal silicate with a modulus in the range from 1.8 to 2.5,
c) up to 2 wt.% phosphonic acid and/or alkali metal phosphonate,
d) up to 50 wt.% alkali metal phosphate, and
e) up to 10 wt.% polymeric polycarboxylate,

the stated quantities relating to the entire washing or cleaning composition. Unless explicitly stated otherwise, this also applies to all the quantities stated below.

In a preferred embodiment of compositions according to the invention, the water-soluble builder block contains at least 2 of components b), c), d) and e) in quantities of greater than 0 wt.%.

With regard to component a), in a preferred embodiment of compositions according to the invention, 15 wt.% to 25 wt.% of alkali metal carbonate, which may at least in part be replaced by alkali metal hydrogencarbonate, and up to 5 wt.% in particular 0.5 wt.% to 2.5 wt.% of citric acid and/or alkali metal citrate are present. In an alternative embodiment of compositions according to the invention, 5 wt.% to 25 wt.%, in particular 5 wt.% to 15 wt.% of citric acid and/or alkali metal citrate and up to 5 wt.%, in particular 1 wt.% to 5 wt.% of alkali metal carbonate, which may at least in part be replaced by alkali metal hydrogencarbonate, are present as component a). If both alkali metal carbonate and alkali metal hydrogencarbonate are present, component a) preferably comprises alkali metal carbonate and alkali metal hydrogencarbonate in a weight ratio of 10:1 to 1.1.

With regard to component b), in a preferred embodiment of compositions according to the invention, 1 wt.% to 5 wt.% of alkali metal silicate with a modulus in the range from 1.8 to 2.5 are present.

With regard to component c), in a preferred embodiment of compositions according to the invention, 0.05wt.% to 1 wt.% of phosphonic acid and/or alkali metal phosphonate are present. Phosphonic acids are here also taken to mean optionally substituted alkylphosphonic acids, which may also comprise two or more phosphonic acid groups ("polyphosphonic acids"). They are preferably selected from hydroxy- and/or aminoalkylphosphonic acids and/or the alkali metal salts thereof, such as for example dimethylaminomethane diphosphonic acid, 3-aminopropyl-1-hydroxy-1,1-diphosphonic acid, 1-amino-1-phenylmethane diphosphonic acid, 1-hydroxyethane-1,1-diphosphonic acid, aminotris(methylenephosphonic acid), N,N,N',N'-ethylenediamine-tetrakis-(methylenephosphonic acid) and acylated derivatives of phosphorous acid, which may also be used in any desired mixtures.

With regard to component d), in a preferred embodiment of compositions according to the invention, 15 wt.% to 35 wt.% of alkali metal phosphate, in particular trisodium polyphosphate, are present. "Alkali metal phosphate" is the summary name for the alkali metal (in particular sodium and potassium) salts of the various phosphoric acids, it being possible to distinguish between meta-phosphoric acids $(HPO_3)_n$ and ortho-phosphoric acid $H_3PO_4$ as well as higher molecular weight representatives. The phosphates here combine a number of advantages: they act as alkalinity donors, prevent lime deposits on machine parts or lime incrustation of fabrics and, moreover, contribute to cleaning performance. Sodium dihydrogenphosphate, $NaH_2PO_4$, exists as a dihydrate (density 1.91 $gcm^{-3}$, melting point 60°C) and as a monohydrate (density 2.04 $gcm^{-3}$). Both salts are white powders, very readily soluble in water, which lose their water of crystallization when heated and at 200°C change into the weakly acidic diphosphate (disodium hydrogendiphosphate, $Na_2H_2P_2O_7$) and at a higher temperature into sodium trimetaphosphate ($Na_3P_3O_9$) and Maddrell's salt. $NaH_2PO_4$ exhibits an acidic reaction; it is obtained when phosphoric acid is adjusted with sodium hydroxide solution to a pH value of 4.5 and the slurry is atomized. Potassium dihydrogenphosphate (primary or monobasic potassium phosphate, potassium diphosphate, KDP), $KH_2PO_4$, is a white salt with a density of 2.33 $gcm^{-3}$, has a melting point of 253°C (decomposition with formation of $(KPO_3)_x$ potassium polyphosphate) and is readily soluble in water. Disodium hydrogenphosphate (secondary sodium phosphate), $Na_2HPO_4$, is a colorless, very readily water-soluble crystalline salt. It exists in anhydrous form and with 2 mol (density 2.066 $gcm^{-3}$, water loss at 95°C), 7 mol (density 1.68 $gcm^{-3}$, melting point 48°C with loss of 5 $H_2O$) and 12 mol of water (density 1.52 $gcm^{-3}$, melting point 35°C with loss of 5 $H_2O$), is anhydrous at 100°C and when heated further changes into the diphosphate $Na_4P_2O_7$. Disodium hydrogenphosphate is produced by neutralizing phosphoric acid with soda solution using phenolphthalein as indicator. Dipotassium hydrogenphosphate (secondary or dibasic potassium phosphate), $K_2HPO_4$, is an amorphous, white salt, which is readily soluble in water. Trisodium phosphate, tertiary sodium phosphate, $Na_3PO_4$, are colorless crystals, which have as dodecahydrate a density of 1.62 $gcm^{-3}$ and a melting point of 73-76°C (decomposition), as decahydrate (corresponding to 19-20% $P_2O_5$) a melting point of 100°C and in anhydrous form (corresponding to 39-40% $P_2O_5$) a density of 2.536 $gcm^{-3}$. Trisodium phosphate is readily soluble in water with an alkaline reaction and is produced by evaporation of a solution of precisely 1 mol of disodium phosphate and 1 mol of NaOH. Tripotassium phosphate (tertiary or tribasic potassium phosphate), $K_3PO_4$, is a white, deliquescent, granular powder with a density of 2.56 $gcm^{-3}$, has a melting point of 1340°C and is readily soluble in water with an alkaline reaction. It arises for example when Thomas slag is heated with carbon and potassium sulfate. Despite their relatively high price, the more readily soluble and therefore highly effective potassium phosphates are often preferred in the cleaning composition industry over corresponding sodium compounds. Tetrasodium diphosphate (sodium pyro-

phosphate), $Na_4P_2O_7$, exists in anhydrous form (density 2.534 $gcm^{-3}$, melting point 988°C, also stated as 880°C) and as decahydrate (density 1.815-1.836 $gcm^{-3}$, melting point 94°C with water loss). Solid substances comprise colorless crystals which are soluble in water with an alkaline reaction. $Na_4P_2O_7$ arises on heating disodium phosphate to >200°C or by reacting phosphoric acid with soda in a stoichiometric ratio and dehydrating the solution by atomization. The decahydrate complexes heavy metal salts and hardness forming substances and therefore reduces the hardness of the water. Potassium diphosphate (potassium pyrophosphate), $K_4P_2O_7$, exists in the form of the trihydrate and is a colorless, hygroscopic powder with a density of 2.33 $gcm^{-3}$, which is soluble in water, the pH value of the 1% solution amounting to 10.4 at 25°C. Condensing $NaH_2PO_4$ or $KH_2PO_4$ gives rise to higher molecular weight sodium and potassium phosphates, in which it is possible to distinguish between cyclic representatives, the sodium or potassium metaphosphates, and chain types, the sodium or potassium polyphosphates The latter in particular have a plurality of names: fused or thermal phosphates, Graham's salt, Kurrol's salt and Maddrell's salt. All higher sodium and potassium phosphates are jointly designated condensed phosphates. The technically important pentasodium triphosphate, $Na_5P_3O_{10}$ (sodium tripolyphosphate), is a non-hygroscopic, white, water-soluble salt, which is anhydrous or crystallized with 6 $H_2O$, of the general formula NaO-[P(O)(ONa)-O]$_n$-Na with n=3. At room temperature approx. 17 g, at 60°C approx. 20 g, at 100°C around 32 g of the salt containing no water of crystallization dissolve in 100 g of water; after heating the solution for two hours to 100°C, approx. 8% orthophosphate and 15% diphosphate are obtained by hydrolysis. When producing pentasodium triphosphate, phosphoric acid is reacted with soda solution or sodium hydroxide solution in a stoichiometric ratio and the solution is dehydrated by atomization.

As with Graham's salt and sodium diphosphate, pentasodium triphosphate dissolves many insoluble metal compounds (even lime soaps etc.). Pentapotassium triphosphate, $K_5P_3O_{10}$ (potassium tripolyphosphate), is commercially available, for example, in the form of a 50 wt.% solution (>23% $P_2O_5$, 25% KaO). Potassium polyphosphates are widely used in the washing and cleaning composition industry. Sodium potassium tripolyphosphates also exist, which may likewise be used for the purposes of the present invention. These arise for example if sodium trimetaphosphate is hydrolyzed with KOH:

$$(NaPO_3)_3 + 2\ KOH \rightarrow Na_3K_2P_3O_{10} + H_2O$$

They may be used according to the invention in exactly the same way as sodium tripolyphosphate, potassium tripolyphosphate or mixtures of these two; mixtures of sodium tripolyphosphate and sodium potassium tripolyphosphate or mixtures of potassium tripolyphosphate and sodium potassium tripolyphosphate or mixtures of sodium tripolyphosphate and potassium tripolyphosphate and sodium potassium tripolyphosphate may also be used according to the invention.

With regard to component e), in a preferred embodiment of compositions according to the invention, 1.5 wt.% to 5wt.% of polymeric polycarboxylate are present, in particular selected from the polymerization or copolymerization products of acrylic acid, methacrylic acid and/or maleic acid. Among these, the homopolymers of acrylic acid are preferred and, among these in turn, those with an average molar mass in the range from 5,000 D to 15,000 D (PA standard) are particularly preferred.

In another preferred embodiment, the detergent composition according to.the invention comprises one or more additional enzymes such as a protease, lipase, cutinase, an amylase, carbohydrase, cellulase, pectinase, mannanase, arabinase, galactanase, xylanase, oxidase, e.g., a laccase, and/or peroxidase.

In general the properties of the selected enzyme(s) should be compatible with the selected detergent, (i.e., pH-optimum, compatibility with other enzymatic, and non-enzymatic ingredients, etc.), and the enzyme(s) should be present in effective amounts.

Cellulases: Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium, e.g.,* the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases having color care benefits. Examples of such cellulases are cellulases described in EP 0.495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and PCT/DK98/00299.

Commercially available cellulases include Celluzyme™, and Carezyme™ (Novozymes A/S), Clazinase™, and Puradax HA™ (Genencor International Inc.), and KAC-500(B)™ (Kao Corporation).

Proteases: Suitable proteases include those of animal, vegetable or microbial origin. Microbial origin is preferred. Chemically modified or protein engineered mutants are included. The protease may be a serine protease or a metalloprotease, preferably an alkaline microbial protease or a trypsin-like protease. Examples of alkaline proteases are subtilisins, especially those derived from *Bacillus, e.g.,* subtilisin Novo, subtilisin Carlsberg, subtilisin 309, subtilisin 147 and

subtilisin 168 (described in WO 89/06279). Examples of trypsin-like proteases are trypsin (e.g., of porcine or bovine origin) and the *Fusarium* protease described in WO 89/06270 and WO 94/25583.

Examples of useful proteases are the variants described in WO 92/19729, WO 98/20115, WO 98/20116, and WO 98/34946, especially the variants with substitutions in one or more of the following positions: 27, 36, 57, 76, 87, 97, 101, 104, 120, 123, 167, 170, 194, 206, 218, 222, 224, 235, and 274.

Preferred commercially available protease enzymes include Alcalase™, Savinase™, Primase™, Duralase™, Esperase™, and Kannase™ (Novozymes A/S), Maxatase™, Maxacal™, Maxapem™, Properase™, Purafect™, Purafect OxP™, FN2™, and FN3™ (Genencor International Inc.).

Lipases and Cutinases: Suitable lipases and cutinases include those of bacterial or fungal origin. Chemically modified or protein engineered mutants are included. Examples include lipase from *Thermomyces, e.g.,* from *T. lanuginosus* (previously named *Humicola lanuginosa*) as described in EP 258 068 and EP 305 216, cutinase from *Humicola, e.g. H. insolens* as described in WO 96/13580, a *Pseudomonas* lipase, e.g., from *P. alcaligenes* or *P. pseudoalcaligenes* (EP 218 272), *P. cepacia* (EP 331 376), *P. stutzeri* (GB 1,372,034), *P. fluorescens, Pseudomonas sp.* strain SD 705 (WO 95/06720 and WO 96/27002), *P. wisconsinensis* (WO 96/12012), a *Bacillus* lipase, *e.g.,* from *B. subtilis* (Dartois et al., 1993, Biochemica et Biophysica Acta, 1131: 253-360), B. *stearothermophilus* (JP 64/744992) or *B. pumilus* (WO 91/16422).

Other examples are lipase variants such as those described in WO 92/05249, WO 94/01541, EP 407 225, EP 260 105, WO 95/35381, WO 96/00292, WO 95/30744, WO 94/25578, WO 95/14783, WO 95/22615, WO 97/04079, WO 97/07202, WO 00/060063, WO2007/087508 and WO 2009/109500.

Preferred commercially available lipase enzymes include Lipolase™, Lipolase Ultra™, and Lipex™; Lecitase™, Lipolex™; Lipoclean™, Lipoprime™ (Novozymes A/S). Other commercially available lipases include Lumafast (Genencor Int Inc); Lipomax (Gist-Brocades/Genencor Int Inc) and Bacillus sp lipase from Solvay.

Amylases: Suitable amylases ($\alpha$ and/or p) include those of bacterial or fungal origin. Chemically modified or protein engineered mutants are included. Amylases include for example, $\alpha$-amylases obtained from *Bacillus, e.g.,* a special strain of *Bacillus licheniformis,* described in more detail in GB 1,296,839.

Examples of useful amylases are the variants described in WO 94/02597, WO 94/18314, WO 96/23873, and WO 97/43424, especially the variants with substitutions in one or more of the following positions: 15, 23, 105, 106, 124, 128, 133, 154, 156, 181, 188, 190, 197, 202, 208, 209, 243, 264, 304, 305, 391, 408, and 444.

Commercially available amylases are Duramyl™, Termamyl™, Fungamyl™ and BAN™ (Novozymes A/S), Rapidase™ and Purastar™ (from Genencor International Inc.).

Peroxidases/Oxidases: Suitable peroxidases/oxidases include those of plant, bacterial or fungal origin. Chemically modified or protein engineered mutants are included. Examples of useful peroxidases include peroxidases from *Coprinus, e.g.,* from C. *cinereus,* and variants thereof as those described in WO 93/24618, WO 95/10602, and WO 98/15257.

Commercially available peroxidases include Guardzyme™ (Novozymes A/S).

The detergent enzyme(s) may be included in a detergent composition by adding separate additives containing one or more enzymes, or,by adding a combined additive comprising all of these enzymes. A detergent additive, *i.e.*, a separate additive or a combined additive, can be formulated, for example, as a granulate, liquid, slurry, etc. Preferred detergent additive formulations are granulates, in particular non-dusting granulates, liquids, in particular stabilized liquids, or slurries.

Non-dusting granulates may be produced, *e.g.,* as disclosed in US 4,106,991 and 4,661,452 and may optionally be coated by methods known in the art. Examples of waxy coating materials are poly(ethylene oxide) products (polyethyleneglycol, PEG) with mean molar weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 238,216.

In another preferred embodiment of the invention, the composition contains 5 wt.% to 50 wt.%, in particular 8-30 wt.% of anionic and/or nonionic surfactant, up to 60 wt.%, in particular 5-40 wt.% of builder substance and 0.2 wt.% to 2 wt.% of enzyme, selected from proteases, lipases, cutinases, amylases, pullulanases, mannanases, cellulases, oxidases and peroxidases and mixtures thereof.

Organic solvents which may be used in the compositions according to the invention, in particular if these are in liquid or pasty form, include alcohols with 1 to 4 C atoms, in particular methanol, ethanol, isopropanol and tert-butanol, diols with 2 to 4 C atoms, in particular ethylene glycol and propylene glycol, and mixtures thereof and the ethers derivable from the stated classes of compounds. Such water-miscible solvents are preferably present in the washing compositions according to the invention in quantities of no more than 30 wt.%, in particular of 6 wt.% to 20 wt.%.

In order to establish a desired pH value which is not automatically obtained by mixing the remaining components, the compositions according to the invention may contain acids which are compatible with the system and are environmentally compatible, in particular citric acid, acetic acid, tartaric acid, malic acid, lactic acid, glycolic acid, succinic acid, glutaric acid and/or adipic acid, as well as mineral acids, in particular sulfuric acid, or bases, in particular ammonium or alkali metal hydroxides. Such pH regulators are present in the compositions according to the invention preferably in an amount of no more than 20 wt.%, in particular of 1.2 wt.% to 17 wt.%.

Polymers with a soil detachment capacity, which are often known as "soil release" active ingredients or, due to their ability to provide a soil-repelling finish on the treated surface, for example the fiber, as "soil repellents," are for example nonionic or cationic cellulose derivatives. Polymers with a soil detachment capacity, in particular with regard to polyesters, include copolyesters prepared from dicarboxylic acids, for example adipic acid, phthalic acid or terephthalic acid, diols, for example ethylene glycol or propylene glycol, and polydiols, for example polyethylene glycol or polypropylene glycol. Polyesters with a soil detachment capacity which are preferably used include those compounds which, in formal terms, are obtainable by esterifying two monomer moieties, the first monomer being a dicarboxylic acid HOOC-Ph-COOH and the second monomer a diol HO-$(CHR^{11}-)_a$OH, which may also be present as a polymeric diol H-$(O-(CHR^{11}-)_a)_b$OH. Ph here means an o-, m- or p-phenylene residue which may bear 1 to 4 substituents selected from alkyl residues with 1 to 22 C atoms, sulfonic acid groups, carboxyl groups and mixtures thereof, $R^{11}$ means hydrogen, an alkyl residue with 1 to 22 C atoms and mixtures thereof, a means a number from 2 to 6 and b a number from 1 to 300. The polyesters obtainable therefrom preferably contain not only monomer diol units -O-$(CHR^{11}-)_a$O- but also polymer diol units -$(O-(CHR^{11}-)_a)_b$O-. The molar ratio of monomer diol units to polymer diol units preferably amounts to 100:1 to 1:100, in particular to 10:1 to 1:10. In the polymer diol units, the degree of polymerization b is preferably in the range from 4 to 200, in particular from 12 to 140. The molecular weight or average molecular weight or the maximum of the molecular weight distribution of preferred polyesters with a soil detachment capacity is in the range from 250 to 100,000, in particular from 500 to 50,000. The acid on which the residue Ph is based is preferably selected from terephthalic acid, isophthalic acid, phthalic acid, trimellitic acid, mellitic acid, the isomers of sulfophthalic acid, sulfoisophthalic acid and sulfoterephthalic acid and mixtures thereof. Where the acid groups thereof are not part of the ester bond in the polymer, they are preferably present in salt form, in particular as an alkali metal or ammonium salt. Among these, sodium and potassium salts are particularly preferred. If desired, instead of the monomer HOOC-Ph-COOH, the polyester with a soil detachment capacity may contain small proportions, in particular no more than 10 mol% relative to the proportion .of Ph with the above-stated meaning, of other acids which comprise at least two carboxyl groups. These include, for example, alkylene and alkenylene dicarboxylic acids such as malonic acid, succinic acid, fumaric acid, maleic acid, glutaric acid, adipic acid, pimelic acid, suberic acid, azelaic acid and sebacic acid. Preferred diols HO-$(CHR^{11}-)_a$OH include those in which $R^{11}$ is hydrogen and a is a number from 2 to 6, and those in which a has the value 2 and $R^{11}$ is selected from hydrogen and alkyl residues with 1 to 10, in particular 1 to 3 C atoms. Among the latter-stated diols, those of the formula HO-$CH_2$-$CHR^{11}$-OH, in der $R^{11}$ has the above-stated meaning, are particularly preferred. Examples of diol components are ethylene glycol, 1,2-propylene glycol, 1,3-propylene glycol, 1,4-butanediol, 1,5-pentanediol, 1,6-hexanediol, 1,8-octanediol, 1,2-decanediol, 1,2-dodecanediol and neopentyl glycol. Among the polymeric diols, polyethylene glycol with an average molar mass in the range from 1000 to 6000 is particularly preferred. If desired, these polyesters may also be end group-terminated, with end groups which may be considered being alkyl groups with 1 to 22 C atoms and esters of monocarboxylic acids: The end groups attached via ester bonds may be based on alkyl, alkenyl and aryl monocarboxylic acids with 5 to 32 C atoms, in particular 5 to 18 C atoms. These include valeric acid, caproic acid, enanthic acid, caprylic acid, pelargonic acid, capric acid, undecanoic acid, undecenoic acid, lauric acid, lauroleic acid, tridecanoic acid, myristic acid, myristoleic acid, pentadecanoic acid, palmitic acid, stearic acid, petroselinic acid, petroselaidic acid, oleic acid, linoleic acid, linolaidic acid, linolenic acid, eleosteatic acid, arachidic acid, gadoleic acid, arachidonic acid, behenic acid, erucic acid, brassidic acid, clupanodonic acid, lignoceric acid, cerotic acid, melissic acid, benzoic acid, which may bear 1 to 5 substituents having a total of up to 25 C atoms, in particular 1 to 12 C atoms, for example tert.-butylbenzoic acid. The end groups may also be based on hydroxymonocarboxylic acids with 5 to 22 C atoms, which for example include hydroxyvaleric acid, hydroxycaproic acid, ricinoleic acid, the hydrogenation product thereof, hydroxystearic acid, and o-, m- and p-hydroxybenzoic acid. The hydroxymonocarboxylic acids may in turn be joined to one another via their hydroxyl group and their carboxyl group and thus be repeatedly present in an end group. The number of hydroxymonocarboxylic acid units per end group, i.e. their degree of oligomerization, is preferably in the range from 1 to 50, in particular from 1 to 10. In a preferred development of the invention, polymers of ethylene terephthalate and polyethylene oxide terephthalate, in which the polyethylene glycol units have molar weights of 750 to 5000 and the molar ratio of ethylene terephthalate to polyethylene oxide terephthalate amounts to 50:50 to 90:10, are used alone or in combination with cellulose derivatives.

Dye transfer inhibitors which may be considered for use in compositions according to the invention for washing textiles include in particular polyvinylpyrrolidones, polyvinylimidazoles, polymeric N-oxides such as poly-(vinylpyridine-N-oxide) and copolymers of vinylpyrrolidone with vinylimidazole and optionally further monomers.

The compositions according to the invention for use in washing textile may contain anticrease compositions

since textile fabrics, in particular made from rayon, wool, cotton and mixtures thereof, may have a tendency to crease, because the individual fibers are sensitive to being bent, kinked, pressed and squashed transversely of the fiber direction. These include for example synthetic products based on fatty acids, fatty acid esters, fatty acid amides, fatty acid alkylol esters, fatty acid alkylol amides or fatty alcohols, which have generally been reacted with ethylene oxide, or products based on lecithin or modified phosphoric acid esters.

Graying inhibitors have the task of keeping dirt which has been dissolved away from the hard surface and in particular from the textile fiber suspended in the liquor. Water-soluble colloids of a mainly organic nature are suitable for this purpose, for example starch, size, gelatin, salts of ether carboxylic acids or ether sulfonic acids of starch or cellulose or salts of acidic sulfuric acid esters of cellulose or starch. Water-soluble polyamides containing acidic groups are also suitable for this purpose. Derivatives of starch other than those stated above, for example aldehyde starches, may further be used. Cellulose ethers, such as carboxymethylcellulose (Na salt), methylcellulose, hydroxyalkylcellulose and mixed ethers, such as methylhydroxyethylcellulose, methylhydroxypropylcellulose, methylcarboxymethylcellulose and mixtures thereof, are preferably used, for example in quantities of 0.1 to 5 wt.% relative to the compositions.

The washing compositions may contain optical brighteners, among these in particular derivatives of diaminostilbene disulfonic acid or the alkali metal salts thereof. Suitable compounds are, for example, salts of 4,4'-bis(2-anilino-4-morpholino-1,3,5-triazinyl-6-amino)stilbene 2,2'-disulfonic acid or compounds of similar structure which, instead of the morpholino group, bear a diethanolamino group, a methylamino group, an anilino group or a 2-methoxyethylamino group. Brighteners of the substituted diphenylstyryl type may furthermore be present, for example the alkali metal salts of 4,4'-bis(2-sulfostyryl)diphenyl, 4,4'-bis(4-chloro-3-sulfostyryl)diphenyl, or 4-(4-chlorostyryl)-4'-(2-sulfostyryl)diphenyl. Mixtures of the above-stated optical brighteners may also be used.

Especially for use in machine washing or cleaning methods, it may be advantageous to add conventional foam inhibitors to the compositions. Suitable foam inhibitors are, for example, soaps of natural or synthetic origin, which comprise an elevated proportion of $C_{18}$-$C_{24}$ fatty acids. Suitable non-surfactant foam inhibitors are, for example, organopolysiloxanes and mixtures thereof with microfine, optionally silanized silica as well as paraffins, waxes, microcrystalline waxes and mixtures thereof with silanized silica or bis-fatty acid alkylenediamides. Mixtures of different foam inhibitors are also advantageously used, for example mixtures of silicones, paraffins or waxes. The foam inhibitors, in particular foam inhibitors containing silicone and/or paraffin, are preferably bound to a granular carrier substance which is soluble or dispersible in water. Mixtures of paraffins and bistearylethylenediamide are particularly preferred here.

Active ingredients for avoiding tarnishing of silver objects or "silver corrosion inhibitors" may moreover be used in compositions according to the invention. Preferred silver anticorrosion compositions are organic disulfides, dihydric phenols, trihydric phenols, optionally alkyl- or aminoalkyl-substituted triazoles such as benzotriazole and cobalt, manganese, titanium, zirconium, hafnium, vanadium or cerium salts and/or complexes, in which the stated metals are present in one of the oxidation states II, III, IV, V or VI.

The protease variant may assume the form of powder or granules, which may also optionally be coated and/or colored and may contain conventional carrier materials and/or granulation auxiliaries. In the case of use in granule form, the granules may if desired also contain further active substances.

In another preferred embodiment of the invention, the detergent composition is a liquid or a solid, preferably a solid which is a powder or is in granular form or is a tablet. In another preferred embodiment, the composition is a machine dishwashing composition in particular for automatic dishwashing which is in tablet form.

The production of solid compositions according to the invention is unproblematic and may proceed in a manner known in principle, for example by spray drying or granulation, with any peroxy compound and bleach-boosting active ingredient optionally being added subsequently. Compositions according to the invention with an elevated bulk density, in particular in the range from 650 g/l to 950 g/l, may preferably be produced by a method comprising an extrusion step. Washing or cleaning compositions or disinfectants according to the invention in the form of aqueous solutions or solutions containing other conventional solvents are particularly advantageously produced by simply mixing the ingredients, which may be introduced into an automatic mixer without solvent or as a solution.

**Methods and Uses**

The present invention is also directed to the use of a detergent composition as described herein in a cleaning process such as laundry and/or hard surface cleaning. Especially, the present invention is directed to the use of a detergent compositions according to the invention in laundry of textile and fabrics, such as house hold laundry washing and industrial laundry washing.

Further, the invention is also directed to the use of a detergent compositions according to the invention in hard surface cleaning such as automated Dish Washing (ADW), car wash and cleaning of Industrial surfaces.

The subtilisin variants of the present invention may be added to and thus become a component of a detergent composition. Thus one aspect of the invention relates to the use of a subtilisin variant, comprising the substitutions corresponding to Y217X and N218Z of SEQ ID NO: 2, wherein X and Z are selected from the groups consisting of D,

E and wherein the variant has at least 90% identity to SEQ ID NO: 2 or 4, and wherein the number of alterations in the subtilisin variant is 1-20, e.g., 1-10 and 1-5, such as 1, 2, 3, 4, 5, 6, 7, 8, 9 or 10 alterations in a cleaning process such as laundry and/or hard surface cleaning. Another aspect relates to the use of a detergent composition comprising a subtilisin variant comprising the substitutions corresponding to Y217X and N218Z of SEQ ID NO: 2, wherein X and Z are selected from the groups consisting of D, E, wherein the variant has a sequence identity to SEQ ID NO: 2 or 4 of at least 90%, at least 91%, at least 92%, at least 93%, at least 94%, at least 95% identity, at least 96%, at least 97%, at least 98%, or at least 99%, but less than 100%, sequence identity to the mature polypeptide of SEQ ID NO: 2 or 4, and wherein the number of alterations in the subtilisin variant is 1-20, *e.g*., 1-10 and 1-5, such as 1, 2, 3, 4, 5, 6, 7, 8, 9 or 10 alterations.

One embodiment of the inventions relates to the use of a of a subtilisin variant, comprising the substitutions corresponding to Y217X and N218Z of SEQ ID NO: 2, wherein X and Z are selected from the groups consisting of D, E and wherein the variant has at least 90%, at least 91%, at least 92%, at least 93%, at least 94%, at least 95% identity, at least 96%, at least 97%, at least 98%, or at least 99%, but less than 100%, sequence identity to the mature polypeptide of SEQ ID NO: 2 or 4, and wherein the number of alterations in the subtilisin variant is 1-20. *e.g*., 1-10 and 1-5, such as 1, 2, 3, 4, 5, 6, 7, 8, 9 or 10 alterations, in a cleaning process such as laundry and/or hard surface cleaning, wherein the variant has increased wash performance relative to the parent or relative to a protease parent having the identical amino acid sequence of said variant but not having the substitutions at one or more of said positions when tested in example 2, as described under "Material and Methods".

The cleaning process or the textile care process may for example be a laundry process, a dishwashing process or cleaning of hard surfaces such as bathroom tiles, floors, table tops, drains, sinks and washbasins. Laundry processes can for examples be household laundering, but it may also be industrial laundering.

Furthermore, the invention relates to a process for laundering of fabrics and/or garments where the process comprises treating fabrics with a washing solution containing a detergent composition according to the invention. The cleaning process or a textile care process can for example be carried out in a machine washing process or in a manual washing process. The washing solution can for example be an aqueous washing solution containing a detergent composition.

The fabrics and/or garments subjected to a washing, cleaning or textile care process of the present invention may be conventional washable laundry, for example household laundry. Preferably, the major part of the laundry is garments and fabrics, including knits, woven, denims, non-woven, felts, yarns, and towelling. The fabrics may be cellulose based such as natural cellulosics, including cotton, flax, linen, jute, ramie, sisal or coir or manmade cellulosics (e.g., originating from wood pulp) including viscose/rayon, ramie, cellulose acetate fibers (tricell), lyocell or blends thereof. The fabrics may also be non-cellulose based such as natural polyamides including wool, camel, cashmere, mohair, rabit and silk or synthetic polymer such as nylon, aramid, polyester, acrylic, polypropylen and spandex/elastane, or blends thereof as well as blend of cellulose based and non-cellulose based fibers. Examples of blends are blends of cotton and/or rayon/viscose with one or more companion material such as wool, synthetic fibers (e.g., polyamide fibers, acrylic fibers, polyester fibers, polyvinyl alcohol fibers, polyvinyl chloride fibers, polyurethane fibers, polyurea fibers, aramid fibers), and cellulose-containing fibers (e.g., rayon/viscose, ramie, flax, linen, jute, cellulose acetate fibers, lyocell).

The last few years there has been an increasing interest in replacing components in detergents, which is derived from petrochemicals with renewable biological components such as enzymes and polypeptides without compromising the wash performance.

The invention further concerns the use of protease variants and/or detergent compositions of the invention in a proteinaceous stain removing processes. The proteinaceous stains may be stains such as food stains, e.g., baby food, sebum, cocoa, egg, blood, milk, ink, grass, or a combination hereof.

Typical detergent compositions include various components in addition to the enzymes, these components have different effects, some components like the surfactants lower the surface tension in the detergent, which allows the stain being cleaned to be lifted and dispersed and then washed away, other components like bleach systems remove discolor often by oxidation and many bleaches also have strong bactericidal properties, and are used for disinfecting and sterilizing. Yet other components like builder and chelator softens, e.g., the wash water by removing the metal ions form the liquid.

In a particular embodiment, the invention concerns the use of a detergent composition according to the invention, i.e. comprising a protease variant as described above, in laundry or dish wash, wherein said detergent composition further comprises at least one or more of the following: a surfactant, a builder, a chelator or chelating agent, bleach system and/or bleach component, in particular a surfactant, a builder, a chelator or chelating agent, bleach system and/or bleach component as described above. In another embodiment of said use, the amount of a surfactant, a builder, a chelator or chelating agent, bleach system and/or bleach component are reduced compared to amount of surfactant, builder, chelator or chelating agent, bleach system and/or bleach component used without the added protease variant of the invention. Preferably at least one component which is a surfactant, a builder, a chelator or chelating agent, bleach system and/or bleach component is present in an amount that is 1% less, such as 2% less, such as 3% less, such as

4% less, such as 5% less, such as 6% less, such as 7% less, such as 8% less, such as 9% less, such as 10% less, such as 15% less, such as 20% less, such as 25% less, such as 30% less, such as 35% less, such as 40% less, such as 45% less, such as 50% less than the amount of the component in the system without the addition of protease variant of the invention, such as a conventional amount of such component. In one aspect, a protease variant of the invention is used in detergent compositions wherein said composition is free of at least one component which is a surfactant, a builder, a chelator or chelating agent, bleach system or bleach component and/or polymer.

**Washing Method**

The detergent compositions of the present invention are ideally suited for use in laundry applications. Accordingly, the present invention includes a method for laundering a fabric. The method comprises the steps of contacting a fabric to be laundered with a cleaning laundry solution comprising the detergent composition according to the invention. The fabric may comprise any fabric capable of being laundered in normal consumer use conditions. The solution preferably has a pH from about 5.5 to about 11.5. The compositions may be employed at concentrations from about 100 ppm, preferably 500 ppm to about 15,000 ppm in solution. The water temperatures typically range from about 5°C to about 95°C, including about 10°C, about 15°C, about 20°C, about 25°C, about 30°C, about 35°C, about 40°C, about 45°C, about 50°C, about 55°C, about 60°C, about 65°C, about 70°C, about 75°C, about 80°C, about 85°C and about 90°C. The water to fabric ratio is typically from about 1:1 to about 30:1.

In particular embodiments, the washing method is conducted at a pH from about 5.0 to about 11.5, or from about 6 to about 10.5, about 5 to about 11, about 5 to about 10, about 5 to about 9, about 5 to about 8, about 5 to about 7, about 5.5 to about 11, about 5.5 to about 10, about 5.5 to about 9, about 5.5 to about 8, about 5.5. to about 7, about 6 to about 11, about 6 to about 10, about 6 to about 9, about 6 to about 8, about 6 to about 7, about 6.5 to about 11, about 6.5 to about 10, about 6.5 to about 9, about 6.5 to about 8, about 6.5 to about 7, about 7 to about 11, about 7 to about 10, about 7 to about 9, or about 7 to about 8, about 8 to about 11, about 8 to about 10, about 8 to about 9, about 9 to about 11, about 9 to about 10, about 10 to about 11, preferably about 5.5 to about 11.5.

In particular embodiments, the washing method is conducted at a degree of hardness of from about 0°dH to about 30°dH, such as about 1°dH, about 2°dH, about 3°dH, about 4°dH, about 5°dH, about 6°dH, about 7°dH, about 8°dH, about 9°dH, about 10°dH, about 11°dH, about 12°dH, about 13°dH, about 14°dH, about 15°dH, about 16°dH, about 17°dH, about 18°dH, about 19°dH, about 20°dH, about 21°dH, about 22°dH, about 23°dH, about 24°dH, about 25°dH, about 26°dH, about 27°dH, about 28°dH, about 29°dH, about 30°dH. Under typical European wash conditions, the degree of hardness is about 16°dH, under typical US wash conditions about 6°dH, and under typical Asian wash conditions, about 3°dH.

The present invention relates to a method of cleaning a fabric, a dishware or hard surface with a detergent composition comprising a protease variant of the invention.

A preferred embodiment concerns a method of cleaning, said method comprising the steps of: contacting an object with a cleaning composition comprising a protease variant of the invention under conditions suitable for cleaning said object In a preferred embodiment the cleaning composition is a detergent composition and the process is a laundry or a dish wash process.

Still another embodiment relates to a method for removing stains from fabric which comprises contacting said a fabric with a composition comprising a protease of the invention under conditions suitable for cleaning said object.

In a preferred embodiment the compositions for use in the methods above further comprise at least one additional enzyme as described above, such as an enzyme selected from the group of hydrolases such as proteases, lipases, cutinases, carbohydrases such as amylases, cellulases, hemicellulases, xylanases, and pectinase or a combination hereof. In yet another preferred embodiment the compositions for use in the methods above comprise a reduced amount of at least one or more of the following components a surfactant, a builder, a chelator or chelating agent, bleach system or bleach component or a polymer.

Also contemplated are compositions and methods of treating fabrics (e.g., to desize a textile) using one or more of the protease of the invention. The protease can be used in any fabric-treating method which is well known in the art (see, e.g., U.S. Patent No. 6,077,316). For example, in one aspect, the feel and appearance of a fabric is improved by a method comprising contacting the fabric with a protease variant of the invention in a solution. In one aspect, the fabric is treated with the solution under pressure.

In one embodiment, the protease variant is applied during or after the weaving of textiles, or during the desizing stage, or one or more additional fabric processing steps. During the weaving of textiles, the threads are exposed to considerable mechanical strain. Prior to weaving on mechanical looms, warp yarns are often coated with sizing starch or starch derivatives in order to increase their tensile strength and to prevent breaking. The protease variant can be applied to remove these sizing protein or protein derivatives. After the textiles have been woven, a fabric can proceed to a desizing stage. This can be followed by one or more additional fabric processing steps. Desizing is the act of removing size from textiles. After weaving, the size coating should be removed before further processing the fabric in

order to ensure a homogeneous and wash-proof result. Also provided is a method of desizing comprising enzymatic hydrolysis of the size by the action of an enzyme.

All issues, subject matter and embodiments which are disclosed for detergent compositions in this application are also applicable for methods and uses described herein. Therefore, it is explicitly referred to said disclosure for the methods and uses described herein as well.

## Examples

Materials and Methods

General molecular biology methods:

Unless otherwise mentioned the DNA manipulations and transformations were performed using standard methods of molecular biology (Sambrook et al. (1989); Ausubel et al. (1995); Harwood and Cutting (1990).

Protease assays:

1) Suc-AAPF-pNA assay:

| | | |
|---|---|---|
| pNA substrate | : | Suc-AAPF-pNA (Bachem L-1400). |
| Temperature | : | Room temperature (25°C) |
| Assay buffers | : | 100mM succinic acid, 100mM HEPES, 100mM CHES, 100mM CABS, 1mM $CaCl_2$, 150mM KCl, 0.01% Triton X-100 adjusted to pH-values 2.0, 3.0, 4.0, 5.0, 6.0, 7.0, 8.0, 9.0, 10.0, and 11.0 with HCl or NaOH. |

20μl protease (diluted in 0.01% Triton X-100) was mixed with 100μl assay buffer. The assay was started by adding 100μl pNA substrate (50mg dissolved in 1.0ml DMSO and further diluted 45x with 0.01% Triton X-100). The increase in $OD_{405}$ was monitored as a measure of the protease activity.

2) Protazyme AK assay:

| | | |
|---|---|---|
| Substrate | : | Protazyme AK tablet (cross-linked and dyed casein; from Megazyme) |
| Temperature | : | 37°C (or set to other assay temperature). |
| Assay buffer | : | 100mM succinic acid, 100mM HEPES, 100mM CHES, 100mM CABS, 1mM $CaCl_2$, 150mM KCl, 0.01% Triton X-100, pH 6.5 or pH 7.0. |

A Protazyme AK tablet was suspended in 2.0ml 0.01% Triton X-100 by gentle stirring. 500μl of this suspension and 500μl assay buffer were dispensed in a microcentrifuge tube and placed on ice. 20μl protease solution (diluted in 0.01% Triton X-100) was added to the ice-cold mixture. The assay was initiated by transferring the tube to a thermomixer at 37°C and shaking at its highest rate (1400 rpm.). After 15 minutes the tube was put back into the ice bath. To remove unreacted substrate, the mixture was centrifuged in an ice cold centrifuge for a few minutes and 200μl supernatant was transferred to a microtiter plate. The absorbance of the supernatant at 650 nm was measured. A sample with 20μl of 0.01% Triton X-100 instead of protease solution was assayed in parallel, and its value was subtracted from the protease sample measurement.

Automatic Mechanical Stress Assay (AMSA) for laundry

In order to assess the wash performance in laundry washing experiments are performed, using the Automatic Mechanical Stress Assay (AMSA). With the AMSA, the wash performance of a large quantity of small volume enzyme-detergent solutions can be examined. The AMSA plate has a number of slots for test solutions and a lid firmly squeezing the laundry sample, the textile to be washed against all the slot openings. During the washing time, the plate, test solutions, textile and lid are vigorously shaken to bring the test solution in contact with the textile and apply mechanical stress in a regular, periodic oscillating manner. For further description see WO02/42740 especially the paragraph "Special method embodiments" on pages 23-24.

The laundry experiments are conducted under the experimental conditions specified below:

| | |
|---|---|
| Detergent dosage | 5 g/L (liquid detergent)<br>2.5 g/L (powder detergent) |
| Test solution volume | 160 micro L |
| pH | As is |
| Wash time | 20 minutes |
| Temperature | 30°C |
| Water hardness | 15°dH |

Model detergents and test materials were as follows:

| | |
|---|---|
| Laundry liquid model detergent | Sodium alkylethoxy sulphate (C-9-15, 2EO) 6.0%<br>Sodium dodecyl benzene sulphonate 3.0%<br>Sodium toluene sulphonate 3.0%<br>Olic acid 2.0%<br>Primary alcohol ethoxylate (C12-15, 7EO) 3.0% |
| | Primary alcohol ethoxylate (C12-15, 3EO) 2.5%<br>Ethanol 0.5%<br>Monopropylene glycol 2.0%<br>Tri-sodium citrate 2H20 4.0%<br>Triethanolamine 0.4%<br>De-ionized water ad 100%<br>pH adjusted to 8.5 with NaOH |
| Laundry powder model detergent | Sodium citrate dehydrate 32.3%<br>Sodium-LAS 24.2%<br>Sodium lauryl sulfate 32.2%<br>Neodol 25-7 (alcohol ethoxylate) 6.4%<br>Sodium sulfate 4.9% |
| Test material | PC-S-37 (whole egg on cotton/polyester)<br>PC-S-39 (aged full egg on cotton/polyester) |

Water hardness was adjusted to 15°dH by addition of $CaCl_2$, $MgCl_2$, and $NaHCO_3$ ($Ca^{2+}$:$Mg^{2+}$ = 4:1:7.5) to the test system. After washing the textiles were flushed in tap water and dried.

The wash performance is measured as the brightness of the colour of the textile washed. Brightness can also be expressed as the intensity of the light reflected from the sample when illuminated with white light. When the sample is stained the intensity of the reflected light is lower, than that of a clean sample. Therefore the intensity of the reflected light can be used to measure wash performance.

Color measurements are made with a professional flatbed scanner (Kodak iQsmart, Kodak, Midtager 29, DK-2605 Brøndby, Denmark), which is used to capture an image of the washed textile.

To extract a value for the light intensity from the scanned images, 24-bit pixel values from the image are converted into values for red, green and blue (RGB). The intensity value (Int) is calculated by adding the RGB values together as vectors and then taking the length of the resulting vector:

$$Int = \sqrt{r^2 + g^2 + b^2}.$$

**Example 1: Preparation and testing of protease variants**

Preparation and Expression of variants

Mutation and introduction of an expression cassette into *Bacillus subtilis.*

All DNA manipulations were done by PCR (e.g. Sambrook et al.; Molecular Cloning; Cold Spring Harbor Laboratory Press) and can be repeated by everybody skilled in the art. Recombinant *B. subtilis* constructs encoding protease variants were used to inoculate shakeflasks containing a rich media (e.g. PS-1: 100 g/L Sucrose (Danisco cat.no. 109-0429), 40 g/L crust soy (soy bean flour), 10g/L $Na_2HPO_4.12H_2O$ (Merck cat.no. 6579), 0.1ml/L Pluronic PE 6100 (BASF 102-3098)). Cultivation typically takes 4 days at 30°C shaking with 220rpm.

Fermentation of variants

Fermentation may be performed by methods well known in the art or as follows. A *B. subtilis* strain harboring the relevant expression plasmid was streaked on a LB-agar plate with a relevant antibiotic (6μg/ml chloramphenicol), and grown overnight at 37°C.

The colonies were transferred to 100 ml PS-1 media supplemented with the relevant antibiotic in a 500 ml shaking flask.

Cells and other undissolved material were removed from the fermentation broth by centrifugation at 4500 rpm for 20-25 minutes. Afterwards the supernatant was filtered to obtain a clear solution.

**Example 2: Testing subtilisin variants**

The wash performance of the protease variants relative to BPN' (SEQ ID NO: 2) with the Y217L mutation were tested in a powder and a liquid model detergent at a temperature of 30°C using the AMSA method as described under "Material and Methods".

Results:

The relative wash performance of the protease variants and their corresponding protease parent (SEQ ID NO: 2) with the Y217L for two stains.PC-S-37 (whole egg on cotton/polyester) and PC-S-39 (Aged full egg on cotton/polyester) are shown in Table 2.1 below.

Table 2.1 AMSA data

| Stain | PC-S-37 | | PC-S-39 |
|---|---|---|---|
| | Det 5 | PDET 2 | PDET 2 |
| Y217L | 100% | 100% | 100% |
| Y217E | 109% | 283% | 133% |
| Y217D | 438% | 700% | 267% |
| Y217R | 120% | 436% | 144% |
| N218E | 648% | 200% | 630% |
| N218D | 335% | 432% | 170% |
| N218S | 631% | - | 830% |
| N218T | 352% | 308% | 520% |
| Y217E N218D | 949% | 693% | 1348% |
| Y217E N218E | 792% | 731% | 1754% |
| Y217K N218K | 582% | 1001% | 160% |
| Y217R N218R | 698% | 634% | 200% |
| Y217R N218K | 569% | 912% | 144% |
| Y217K N218R | 655% | 859% | - |

(continued)

| Stain | PC-S-37 | | PC-S-39 |
|---|---|---|---|
| | Det 5 | PDET 2 | PDET 2 |
| Y217E N218S | 448% | 660% | 827% |
| Y217E N218T | 137% | 511% | - |
| Y217D N218S | 796% | 732% | 2069% |
| Y217D N218R | 10% | 318% | 213% |
| Y217E N218K | 2% | 148% | 8% |

The results show that a negative charge at position 217 or 218, which can be provided by residue D or, E increases performance compared to a variant with no charge at one of these positions i.e. Y217L.

The effect of a negative charge at position 217 or 218 can be further increased by offering a second negative charge which could either the same amino acid or another charged amino acid i.e. either D or E as an example Y217E+N218D and Y217E+N218E compared to the corresponding single mutation variants Y217E, N218D and N218E in the same detergent.

Variants with two negative charges at positions 217 and 218 were better than variants that combined a negative with a positive charge (Y217D+N218R, Y217E+N218K).

From these results it is evident that detergent compositions according to the invention, i.e. comprising variants as described, are advantageous.

The invention described and claimed herein is not to be limited in scope by the specific aspects herein disclosed, since these aspects are intended as illustrations of several aspects of the invention. Any equivalent aspects are intended to be within the scope of this invention. Indeed, various modifications of the invention in addition to those shown and described herein will become apparent to those skilled in the art from the foregoing description. Such modifications are also intended to fall within the scope of the appended claims. In the case of conflict, the present disclosure including definitions will control.

## Claims

1. A detergent composition comprising a subtilisin variant having at least 90%, at least 95%, at least 96%, at least

97%, at least 98%, or at least 99%, but less than 100% sequence identity to the mature polypeptide of SEQ ID NO: 2 or 4 and further comprising the substitutions corresponding to Y217X and N218Z of SEQ ID NO: 2, wherein X and Z are selected from the group consisting of D and E, and wherein the number of alterations in the subtilisin variant is 1-20, *e.g.,* 1-10 and 1-5, such as 1, 2, 3, 4, 5, 6, 7, 8, 9 or 10 alterations.

**2.** The detergent composition according to claim 1 wherein X and Z in the variant are the same amino acid.

**3.** The detergent composition of any of claims 1-2, wherein the variant has an improved wash performance on egg stains compared to the parent or compared to a protease with SEQ ID NO: 2.

**4.** The detergent composition of any of claims 1-3, wherein the composition is a liquid or a solid, preferably a solid which is a powder or is in granular form or is a tablet.

**5.** The detergent composition of any of claims 1-4, wherein the composition is a laundry detergent composition or a dishwashing composition, preferably a machine dishwashing composition.

**6.** The use of a detergent composition according to any of claim 1-5 in a cleaning process.

**7.** The use according to claim 6 wherein the cleaning process is laundry.

**8.** The use according to claim 6 wherein the cleaning process is hard surface cleaning such as dish wash.

**9.** The use of a detergent composition according to any of claims 1-5 for removal of egg stains.

**10.** A method for producing a detergent composition which comprises the step of adding a subtilisin variant which was obtained by a method comprising introducing into a parent subtilisin having at least 90%, at least 95%, at least 96%, at least 97%, at least 98%, at least 99% or 100% sequence identity to the mature polypeptide of SEQ ID NO: 2 or 4 the substitutions corresponding to Y217X and N218Z of SEQ ID NO: 2, wherein X and Z are selected from the group consisting of D and E, and wherein the number of alterations in the subtilisin variant is 1-20, e.g., 1-10 and 1-5, such as 1, 2, 3, 4, 5, 6, 7, 8, 9 or 10 alterations.

**11.** The method of claim 10, wherein the parent subtilisin is selected from the group consisting of:

a. a polypeptide having at least 90% sequence identity to the mature polypeptide of SEQ ID NO: 2 or4;
b. a polypeptide encoded by a polynucleotide having at least 90% identity to the mature polypeptide coding sequence of SEQ ID NO: 1 or 3, or the cDNA sequence thereof.

## Patentansprüche

**1.** Detergenszusammensetzung, die eine Subtilisinvariante mit einer zumindest 90%igen, zumindest 95 %igen, zumindest 96%igen, zumindest 97%igen, zumindest 98%igen oder zumindest 99%igen, aber weniger als 100%igen Sequenzidentität mit dem reifen Polypeptid von SEQ ID NO: 2 oder 4 umfasst und des Weiteren die Substitutionen umfasst, die Y217X und N218Z von SEQ ID NO: 2 entsprechen, wobei X und Z aus der Gruppe ausgewählt sind, bestehend aus D und E, und wobei die Anzahl von Veränderungen in der Subtilisinvariante 1 bis 20 ist, z. B. 1 bis 10 und 1 bis 5, wie z. B. 1, 2, 3, 4, 5, 6, 7, 8, 9 oder 10 Veränderungen, ist.

**2.** Detergenszusammensetzung nach Anspruch 1, wobei X und Z in der Variante die gleiche Aminosäure sind.

**3.** Detergenszusammensetzung nach einem der Ansprüche 1 bis 2, wobei die Variante im Vergleich zum Stammmittel oder im Vergleich zu einer Protease mit der SEQ ID NO: 2 eine verbesserte Waschleistung in Bezug auf Eierflecken aufweist.

**4.** Detergenszusammensetzung nach einem der Ansprüche 1 bis 3, wobei die Zusammensetzung eine Flüssigkeit oder ein Feststoff ist, vorzugsweise ein Feststoff, der ein Pulver ist oder in Granulatform vorliegt oder eine Tablette ist.

**5.** Detergenszusammensetzung nach einem der Ansprüche 1 bis 4, wobei die Zusammensetzung eine Waschmittelzusammensetzung oder eine Geschirrspülzusammensetzung ist, vorzugsweise eine Geschirrspülmaschinenzusammensetzung.

6. Verwendung einer Detergenszusammensetzung nach einem der Ansprüche 1 bis 5 in einem Reinigungsprozess.

7. Verwendung nach Anspruch 6, wobei der Reinigungsprozess ein Waschprozess ist.

8. Verwendung nach Anspruch 6, wobei der Reinigungsprozess die Reinigung einer harten Oberfläche, wie z. B. Geschirrspülen, ist.

9. Verwendung einer Detergenszusammensetzung nach einem der Ansprüche 1 bis 5 zum Entfernen von Eierflecken.

10. Verfahren zum Herstellen einer Detergenszusammensetzung, das den Schritt des Hinzufügens einer Subtilisinvariante umfasst, die mithilfe eines Verfahrens erhalten wurde, umfassend das Einbringen in ein Ausgangssubtilisin mit einer zumindest 90%igen, zumindest 95 %igen, zumindest 96%igen, zumindest 97%igen, zumindest 98%igen oder zumindest 99%igen oder 100%igen Sequenzidentität mit dem reifen Polypeptid von SEQ ID NO: 2 oder 4 umfasst, wobei die Substitutionen Y217X und N218Z von SEQ ID NO: 2 entsprechen, wobei X und Z aus der Gruppe ausgewählt sind, bestehend aus D und E, und wobei die Anzahl von Veränderungen in der Subtilisinvariante 1 bis 20 ist, z. B. 1 bis 10 und 1 bis 5, so wie 1, 2, 3, 4, 5, 6, 7, 8, 9 oder 10 Veränderungen.

11. Verfahren nach Anspruch 10, wobei das Ausgangssubtilisin aus der Gruppe ausgewählt ist, bestehend aus:

a. einem Polypeptid mit einer zumindest 90%igen Sequenzidentität mit dem reifen Polypeptid von SEQ ID NO: 2 oder 4;
b. einem Polypeptid, das von einem Polynukleotid mit einer zumindest 90%igen Identität mit der codierenden Sequenz des reifen Polypeptids von SEQ ID NO: 1 oder 3 oder der cDNA-Sequenz davon codiert wird.

**Revendications**

1. Composition détergente comprenant un variant de la subtilisine ayant une identité de séquence d'au moins 90%, d'au moins 95%, d'au moins 96%, d'au moins 97%, d'au moins 98% ou d'au moins 99%, mais de moins de 100% avec le polypeptide mature de SEQ ID NO: 2 ou 4 et comprenant en outre les substitutions correspondant à Y217X et N218Z de SEQ ID NO: 2, X et Z étant choisis dans le groupe comportant D et E, et le nombre de modifications dans le variant de la subtilisine étant de 1 à 20, par exemple, de 1 à 10 et de 1 à 5, tel que 1, 2, 3, 4, 5, 6. 7, 8, 9 ou 10 altérations.

2. Composition détergente selon la revendication 1, dans laquelle X et Z dans le variant sont les mêmes acides aminés.

3. Composition détergente selon l'une quelconque des revendications 1 ou 2, dans laquelle le variant a une performance de lavage améliorée vis-à-vis des taches d'oeuf par rapport au parent ou par rapport à une protéase ayant SEQ ID NO: 2.

4. Composition détergente de l'une quelconque des revendications 1 à 3, dans laquelle la composition est une matière liquide ou solide, de préférence une matière solide qui se présente sous la forme d'une poudre ou de granulés ou de comprimés.

5. Composition détergente de l'une quelconque des revendications 1 à 4, dans laquelle la composition est une composition détergente pour le lavage du linge ou une composition pour le lavage de la vaisselle, de préférence une composition pour le lavage de la vaisselle en machine.

6. Utilisation d'une composition détergente selon l'une quelconque des revendications 1 à 5 dans un procédé de nettoyage.

7. Utilisation selon la revendication 6, dans laquelle le processus de nettoyage est le lavage du linge.

8. Utilisation selon la revendication 6, dans laquelle le processus de nettoyage est le nettoyage de surfaces dures telles que le lavage de la vaisselle.

**9.** Utilisation d'une composition détergente selon l'une quelconque des revendications 1 à 5 pour l'élimination des taches d'oeuf.

**10.** Procédé de production d'une composition détergente qui comprend l'étape consistant à ajouter un variant de la subtilisine qui a été obtenu par un procédé comprenant l'introduction dans une subtilisine mère ayant une identité de séquence d'au moins 90%, au moins 95%, au moins 96%, au moins 97%, au moins 98%, au moins 99% ou de 100% avec le polypeptide mature de SEQ ID NO: 2 ou 4, les substitutions correspondant à Y217X et N218Z de SEQ ID NO: 2, X et Z étant choisis dans le groupe comportant D et E, et le nombre de modifications dans le variant de la subtilisine étant de 1 à 20, par exemple de 1 à 10 et de 1 à 5, tel que 1, 2, 3, 4, 5, 6, 7, 8, 9 ou 10 altérations.

**11.** Procédé selon la revendication 10, dans lequel la subtilisine mère est choisie dans le groupe comportant :

a. un polypeptide ayant une identité de séquence d'au moins à 90% avec le polypeptide mature de SEQ ID NO: 2 ou 4 ;
b. un polypeptide codé par un polynucléotide ayant une identité d'au moins 90% avec la séquence de codage du polypeptide mature de SEQ ID NO: 1 ou 3, ou la séquence d'ADNc de celui-ci.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 04041979 A
- WO 9402618 A
- WO 0144452 A
- US 20040171154 A
- WO 9517413 A
- WO 9522625 A
- US 5223409 A
- WO 9206204 A
- WO 9219709 A
- WO 9219708 A
- WO 2005105826 A
- WO 2009118375 A
- DE 2412837
- US 4435307 A
- US 5648263 A
- US 5691178 A
- US 5776757 A
- WO 8909259 A
- EP 0495257 A
- EP 0531372 A
- WO 9611262 A
- WO 9629397 A
- WO 9808940 A
- WO 9407998 A
- EP 0531315 A
- US 5457046 A
- US 5686593 A
- US 5763254 A
- WO 9524471 A
- WO 9812307 A
- DK 9800299 W
- WO 8906279 A
- WO 8906270 A
- WO 9425583 A
- WO 9219729 A
- WO 9820115 A
- WO 9820116 A
- WO 9834946 A
- EP 258068 A
- EP 305216 A
- WO 9613580 A
- EP 218272 A
- EP 331376 A
- GB 1372034 A
- WO 9506720 A
- WO 9627002 A
- WO 9612012 A
- JP 64744992 B
- WO 9116422 A
- WO 9205249 A
- WO 9401541 A
- EP 407225 A
- EP 260105 A
- WO 9535381 A
- WO 9600292 A
- WO 9530744 A
- WO 9425578 A
- WO 9514783 A
- WO 9522615 A
- WO 9704079 A
- WO 9707202 A
- WO 00060063 A
- WO 2007087508 A
- WO 2009109500 A
- GB 1296839 A
- WO 9402597 A
- WO 9418314 A
- WO 9623873 A
- WO 9743424 A
- WO 9324618 A
- WO 9510602 A
- WO 9815257 A
- US 4106991 A
- US 4661452 A
- GB 1483591 A
- EP 238216 A
- US 6077316 A
- WO 0242740 A


### Non-patent literature cited in the description


- Enzyme Nomenclature 1992. Eur. J. Biochem. Academic Press, 1994, vol. 223, 1-5
- *Eur. J. Biochem.,* 1996, vol. 232, 1-6
- *Eur. J. Biochem.,* 1996, vol. 237, 1-5
- *Eur. J. Biochem.,* 1997, vol. 250, 1-6
- *Eur. J. Biochem.,* 1999, vol. 264, 610-650
- **NIELSEN et al.** *Protein Engineering,* 1997, vol. 10, 1-6
- **NEEDLEMAN ; WUNSCH.** *J. Mol. Biol.,* 1970, vol. 48, 443-453

- **RICE et al.** EMBOSS: The European Molecular Biology Open Software Suite. *Trends Genet.,* 2000, vol. 16, 276-277
- **RICE et al.** *EMBOSS: The European Molecular Biology Open Software Suite,* 2000
- **RICE et al.** EMBOSS: The European Molecular Biology Open Software Suite. *Trends Genet,* 2000, vol. 16, 276-277
- **EDGAR.** *Nucleic Acids Research,* 2004, vol. 32, 1792-1797
- **KATOH ; KUMA.** *Nucleic Acids Research,* 2002, vol. 30, 3059-3066
- **KATOH et al.** *Nucleic Acids Research,* 2005, vol. 33, 511-518
- **KATOH ; TOH.** *Bioinformatics,* 2007, vol. 23, 372-374
- **KATOH et al.** *Methods in Molecular Biology,* 2009, vol. 537, 39-64
- **KATOH ; TOH.** *Bioinformatics,* 2010, vol. 26, 1899-1900
- **THOMPSON et al.** *Nucleic Acids Research,* 1994, vol. 22, 4673-4680
- **LINDAHL ; ELOFSSON.** *J. Mol. Biol,* 2000, vol. 295, 613-615
- **ATSCHUL et al.** *Nucleic Acids Res.,* 1997, vol. 25, 3389-3402
- **JONES.** *J. Mol. Biol,* 1999, vol. 287, 797-815
- **MCGUFFIN ; JONES.** *Bioinformatics,* 2003, vol. 19, 874-881
- **GOUGH et al.** *J. Mol. Biol,* 2000, vol. 313, 903-919
- **HOLM ; SANDER.** *Proteins,* 1998, vol. 33, 88-96
- **SHINDYALOV ; BOURNE.** *Protein Engineering,* 1998, vol. 11, 739-747
- **HOLM ; PARK.** *Bioinformatics,* 2000, vol. 16, 566-567
- **SIEZEN et al.** *Protein Engng.,* 1991, vol. 4, 719-737
- **H. NEURATH ; R.L. HILL.** The Proteins. Academic Press, 1979
- **CUNNINGHAM ; WELLS.** *Science,* 1989, vol. 244, 1081-1085
- **HILTON et al.** *J. Biol. Chem.,* 1996, vol. 271, 4699-4708
- **DE VOS et al.** *Science,* 1992, vol. 255, 306-312
- **SMITH et al.** *J. Mol. Biol.,* 1992, vol. 224, 899-904
- **WLODAVER et al.** *FEBS Lett,* 1992, vol. 309, 59-64
- **WALSH.** Enzymatic Reaction Mechanisms. W.H. Freeman and Company, 1979
- **WHITE ; HANDLER ; SMITH.** Principles of Biochemistry. McGraw-Hill Book Company, 1973, 271-272
- **PRIEST.** *Bacteriological Rev.,* 1977, vol. 41, 711-753
- **SIEZEN et al.** *Protein Science,* 1997, vol. 6, 501-523
- **J.E. NESS et al.** *Nature Biotechnology,* 1999, vol. 17, 893-896
- **SIEZEN et al.** *Protein Engng,* 1991, vol. 4, 719-737
- **NILSSON et al.** *EMBO J.,* 1985, vol. 4, 1075
- **NILSSON et al.** *Methods Enzymol.,* 1991, vol. 198, 3
- **FORD et al.** *Protein Expression and Purification,* 1991, vol. 2, 95-107
- **COOPER et al.** *EMBO J.,* 1993, vol. 12, 2575-2583
- **DAWSON et al.** *Science,* 1994, vol. 266, 776-779
- **MARTIN et al.** *J. Ind. Microbiol. Biotechnol,* 2003, vol. 3, 568-576
- **SVETINA et al.** *J. Biotechnol.,* 2000, vol. 76, 245-251
- **RASMUSSEN-WILSON et al.** *Appl. Environ. Microbiol.,* 1997, vol. 63, 3488-3493
- **WARD et al.** *Biotechnology,* 1995, vol. 13, 498-503
- **CONTRERAS et al.** *Biotechnology,* 1991, vol. 9, 378-381
- **EATON et al.** *Biochemistry,* 1986, vol. 25, 505-512
- **COLLINS-RACIE et al.** *Biotechnology,* 1995, vol. 13, 982-987
- **CARTER et al.** *Proteins: Structure, Function, and Genetics,* 1989, vol. 6, 240-248
- **STEVENS.** *Drug Discovery World,* 2003, vol. 4, 35-48
- **SCHERER ; DAVIS.** *Proc. Natl. Acad. Sci. USA,* 1979, vol. 76, 4949-4955
- **BARTON et al.** *Nucleic Acids Res.,* 1990, vol. 18, 7349-4966
- **STORICI et al.** *Nature Biotechnol.,* 2001, vol. 19, 773-776
- **KREN et al.** *Nat. Med.,* 1998, vol. 4, 285-290
- **CALISSANO ; MACINO.** *Fungal Genet. Newslett,* 1996, vol. 43, 15-16
- **TIAN et al.** *Nature,* 2004, vol. 432, 1050-1054
- **REIDHAAR-OLSON ; SAUER.** *Science,* 1988, vol. 241, 53-57
- **BOWIE ; SAUER.** *Proc. Natl. Acad. Sci. USA,* 1989, vol. 86, 2152-2156
- **LOWMAN et al.** *Biochemistry,* 1991, vol. 30, 10832-10837
- **DERBYSHIRE et al.** *Gene,* 1986, vol. 46, 145
- **NER et al.** *DNA,* 1988, vol. 7, 127
- **NESS et al.** *Nature Biotechnology,* 1999, vol. 17, 893-896
- **A. G. GORNALL ; C. S. BARDAWILL ; M.M. DAVID.** *J. Biol. Chem.,* 1948, vol. 177, 751-766
- **DARTOIS et al.** *Biochemica et Biophysica Acta,* 1993, vol. 1131, 253-360
- *Special method embodiments,* 23-24

- **SAMBROOK et al.** Molecular Cloning. Cold Spring Harbor Laboratory Press